# EUROPEAN PATENT SPECIFICATION

(11) **EP 1 549 679 B1**
(45) Date of publication and mention of the grant of the patent: **29.07.2009**
(21) Application number: 03760110.1
(22) Date of filing: 21.05.2003
(51) Int. Cl.: C07K 19/00, C07K 14/435, A61K 38/17, A61P 35/00

(54) **FUSION PROTEIN COMPRISING ANGIOPOIETIN RECEPTOR-BINDING AND A MULTIMERIZATION DOMAIN**
FUSIONSPROTEIN MIT ANGIOPOIETINREZEPTORBINDUNG UND EINER MULTIMERISATIONSDOMÄNE
PROTEINE DE FUSION COMPRENANT UN DOMAINE SE LIANT AU RECEPTEUR DE L'ANGIOPOIETINE ET UN DOMAINE DE MULTIMERISATION

(30) Priority: 21.05.2002 US 382541 P; 18.10.2002 US 273180
(43) Date of publication of application: 06.07.2005
(73) Proprietor: GenExel-Sein, Inc., Yuseong-gu Daejeon 305-701 (KR)
(72) Inventor: Koh, Gou Young Biomedical Research Center, Daejon 305-701 (KR)
(74) Representative: Fiener, Josef
(86) International application number: PCT/IB2003/003814
(87) International publication number: WO 2003/106501

(56) References cited:
- WO-A1-00/37642
- WO-A1-96/16989
- WO-A1-02/070725
- WO-A2-01/47951
- WO-A2-02/02746
- WO-A2-03/048185
- CHO CHUNG-HYUN ET AL: "Long-term and sustained COMP-Ang1 induces long-lasting vascular enlargement and enhanced blood flow" CIRCULATION RESEARCH, vol. 97, no. 1, July 2005 (2005-07), pages 86-94, XP002375752 ISSN: 0009-7330
- CHO CHUNG-HYUN ET AL: "Designed angiopoietin-1 variant, COMP-Ang1, protects against radiation-induced endothelial cell apoptosis" PROCEEDINGS OF THE NATIONAL ACADEMY OF SCIENCES OF THE UNITED STATES OF AMERICA, vol. 101, no. 15, 13 April 2004 (2004-04-13), pages 5553-5558, XP002375753 ISSN: 0027-8424
- PROCOPIO W.N. ET AL.: 'Angiopoietin-1 and -2 coiled coil domains mediate distinct homo-oligomerization patterns, but fibrinogen-like domains mediate ligand activity' JOURNAL OF BIOLOGICAL CHEMISTRY vol. 274, no. 12, 1999, pages 30196 - 30201, XP008052333
- DAVIS S. ET AL.: 'Angiopoietins have distinct modular domains essential for receptor binding, dimerization and superclustering' NATURE STRUCTURAL BIOLOGY vol. 10, no. 1, January 2003, pages 38 - 44, XP008052277

## Description

### BACKGROUND OF THE INVENTION

### 1. Field of the Invention:

The present invention provides for a novel method of producing novel chimeric molecules that have enhanced biological activity, and easier production and purification processes as compared with molecules in their native form. The invention also provides for nucleic acids useful for producing biologically active chimeric polypeptides, and the fusion polypeptides themselves.

2. Description of the Background:

Transmembrane protein kinases serve as signaling receptors for a variety of polypeptide ligands, eliciting such diverse responses as cell survival, proliferation and differentiation from many cell types and tissues (van der Geer et al., 1994, Annu Rev Cell Biol. 10:251-337). Receptor tyrosine kinases (RTKs) have the ability to interact with different ligands and bring about various cellular responses. One type of RTK is tyrosine kinase with immunoglobulin and epidermal growth factor receptor homology domains (Tie), Tie2 (Dumont et al., 1993, Oncogene 8:1293-1301; Mustonen and Alitalo, 1995, J Cell Biol. 129:895-898). Tie2 is expressed predominantly on endothelial cells, hematopoietic cells, or their embryonic precursors, and it is required for normal vascular development (Sato et al., 1995, Nature 376:70-74). Functional disruption of Tie2 in transgenic mice results in embryonic lethality by day E9.5 to 10.5, with effects on the microvasculature resulting in reduced numbers of endothelial cells, and abnormalities of vascular morphogenesis and hematopoiesis (Sato et al., 1995, Nature 376:70-74). Thus, Tie2 is critical for angiogenesis and hematopoiesis during development

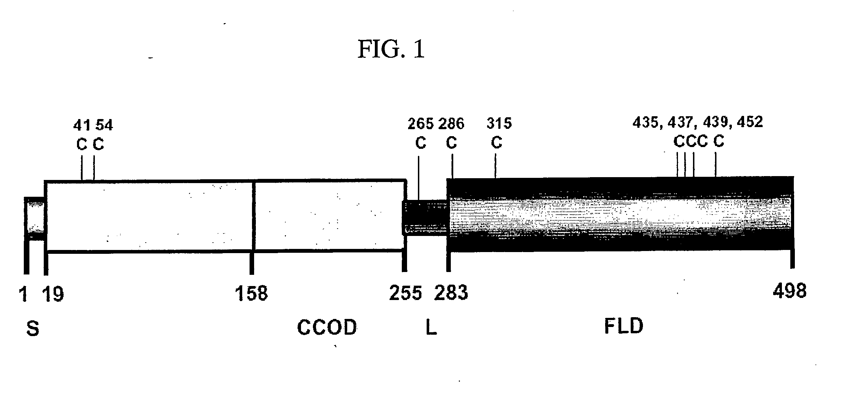

Davis et al. discovered that Ang1 is the ligand for Tie2 (Davis et al., 1996, Cell 87:1161-1169; WO9611269) (Figure 1). Ang1 contains 498 amino acids, including an amino-terminal secretory signal sequence (Figure 1). Human and mouse Ang1 are 97.6% identical. The amino-terminal region, consisting of residues 100-280, is weakly related to myosin and its relatives, in the regions of these proteins where they are known to possess coiled-coil quaternary structure (Figure 1). The second region, consisting of residues 280-498, has strong similarity to the carboxy-terminal domain of fibrinogen (Figure 1). Ang1 is a multimer, held together by coiled-coil structures and disulfide crosslinks. Recombinant Ang1 is a 70-kDa (reduced condition) secreted glycoprotein that binds to the Tie2 receptor with a Kd of approximately 3.7 nM, and induces tyrosine phosphorylation of Tie2 in endothelial cells (Davis et al., 1996, Cell 87:1161-1169).

According to recent findings, Ang1 protein consists of an amino-tenninal secretory signal sequence (residues 1-19), a unique domain (residues 20-158), a coiled-coil oligomerization domain (residues 159-254), a short linker region (residues 255-283), and a carboxy-terminal fibrinogen-like domain (residues 284-498).

Angiopoietin-1 (Ang1) is a specific and critical growth factor for blood vessel formation (Davis et al., 1996, Cell 87:1161-1169; Yancopoulos et al., 2000, Nature 407:242-248). Recent studies indicate that Ang1 could be used for preventing vascular leakages, therapeutic vasculogenesis, and therapeutic endothelial cell survival (Thurston et al., 2000, Nat. Med. 6:460-463; Chae et al., 2000, Arterioscler. Thromb. Vasc. Biol. 20:2573-2578; Kwak et al., 2000, Circulation 101:2317-2324). However, Ang1 protein is not easily available, and generation of recombinant Ang1 is extremely difficult with current techniques. Multimerization of the coiled coil domains during production of the ligand hampered purification.

Complementary DNAs encoding angiopoietin-2 (Ang2) were isolated by low-stringency screening of a cDNA library by using Ang1 cDNA as a probe (Maisonpierre et al., 1997, Science 277:55-60). Ang2 contains 496 amino acids and has a secretory signal sequence. Human and mouse Ang2 are 85% identical and approximately 60% identical to Ang1. Like Ang1, Ang2 has an amino-terminal coiled-coil domain and a carboxy-terminal fibrinogen-like domain. Ang1 and Ang2 have similar binding affinities for Tie2. Ang2 acts as an antagonist of Tie2 through inhibition of Ang1-induced phosphorylation of Tie2 (Maisonpierre et al., 1997, Science 277:55-60). Mouse angiopoietin-3 (Ang3) and human angiopoietin-4 (Ang4) were identified through low stringency hybridization screening with Ang1 and Ang2 cDNAs (Valenzuela et al., 1999, Proc Natl Acad Sci. 96:1904-1909). Ang3 and Ang4 are probably interspecies orthologs. Ang4 phosphorylates Tie2, while Ang3 inhibits Angl-induced phosphorylation of Tie2 (Valenzuela et al., 1999, Proc Natl Acad Sci. 96:1904-1909).

Multimeric form of Ang1 phosphorylates Tie2. In turn, phosphorylated Tie2 interacts cytoplasmically with Grb2, Grb7, Grb14, the protein tyrosine phosphatase Shp2, and the p85 subunit of phosphatidylinositol 3'-kinase (PI 3'-kinase) via their SH2 domains (Jones et al., 1999, J Biol Chem. 274:30896-30905). Association between p85 and Tie2 results in PI 3'-kinase activation and subsequent induction of the serine-threonine kinase Akt. (Kontos et al., 1998, Mol Cell Biol. 18:4131-4140). Ang1 induces endothelial cell survival through this PI 3'-kinase/Akt signaling pathway (Kim et al., 2000, Circ Res. 86:24-29). In addition, Ang1 induces endothelial cell sprouting through the activation of PI 3'-kinase and focal adhesion kinase (Kim et al., 2000, Circ Res. 86:952-959). Thus, Tie2, PI 3'-kinase, Akt, and focal adhesion kinase are crucial elements in the signal transduction pathway leading to survival and migration in endothelial cells. Phosphorylated Tie2 also interacts with Dok-R/Dok-2, leading to activation of Dok-R/Dok-2 (Jones and Dumont, 1998, 17:1097-1108). Phosphorylated Dok-R interacts with rasGAP, Nck, and Crk (Jones and Dumont, 1998, 17:1097-1108). These signaling molecules may be involved in cell migration and proliferation, organization of the cytoskeleton, and regulation of Ras signaling. Recently, it was shown that Dok-R/Dok-2 is responsible for recruiting Nck and p21-activating kinase (Pak/Pak1) to the activated receptor (Master et al., 2001, EMBO J. 20:5919-5928). Localization of this Dok-R-Nck-Pak complex to the activated Tie2 at the cellular membrane is coincident with activation of Pak kinase (Master et al., 2001, EMBO J. 20:5919-5928). This signal transduction pathway may be involved in Angl-mediated migration in endothelial cells. Signal transducers and activators of transcription (STATs) were also found to be potential targets of Tie2 activation (Korpelainen et al., 1999, Oncogene, 18:1-8). Phosphorylated Tie2, in turn, activates STAT3 and STAT5 (Korpelainen et al., 1999, Oncogene, 18:1-8). Since STAT3 and/or STAT5 are known to be involved in the regulation of cell proliferation, differentiation, migration, and survival in many biological systems, it is possible that some of the Tie2 functions in endothelial cells may be controlled through STAT pathway.

It is known that the Tie2 receptor is expressed not only in endothelial cells but also in hematopoietic stem cells (HSCs), indicating another possible role of Ang1 and Tie2 in hematopoiesis (Iwama et al., 1993, Biochem Biophys Res Commun 195:301-309). In fact, Tie2 deficient mice show severely impaired hematopoiesis (Sato et al., 1995, Nature 376:70-74). And HSCs closely adhere to endothelial cells at several sites in the embryo. Furthermore, it has been found that HSCs produce Ang1, suggesting that HSCs can promote the migration of endothelial cells and establish a hematopoietic environment (Takakura et al., 2000, Cell 102:199-209).

Ang1*, made by Regeneron Pharmaceuticals, Inc., is a recombinant version of Ang1 that is easier to produce and purify (Maisonpierre et al., 1997, Science 277:55-60; (PCT WO 98/05779)). Ang1* contains a modified amino-terminus and mutated Cys²⁶⁵. The biological activity of recombinant Ang1 and Ang1* is similar. However, some of the same problems are encountered in producing Ang1* because the size of Ang1* is too large for efficient recombinant generation.

Both native Ang1 and Ang1* require extensive, expensive and labor-intensive purification schemes that result in relatively poor yields of recombinant protein. The need for cost-effective, simple purification schemes for biologicals intended for clinical use cannot be over-emphasized.

U. S. Patent No. 6,455,035 discloses a method of decreasing or inhibiting vascular permeability in a mammal by administering to the mammal a Tie-2 receptor activator. Moreover, USP '035 also discloses that the coiled coil domain of Ang1 was deleted so that the multimerization of the coiled coil domains would not hamper ligand purification.

WO 00/37462 discloses a method of enhancing the biological activity of Ang1 through deletion of coiled-coil domain and insertion with Fc-portion of immunoglobulin for making a tetramer form of chimeric fusion Ang1 (Ang1-1FD-Fc-FD). However, WO 00/37462 discloses that Ang1-1FD-Fc-FD is equivalent to Ang1* in its ability to stimulate phosphorylation of the Tie2 receptor.

Therefore, there is a need in the art to make a modified ligand molecule that is soluble and easily produced, while having substantially similar or greater potency as the native molecule.

### SUMMARY OF THE INVENTION

The claimed invention overcomes the above-mentioned problems, and provides for novel, biologically active, soluble forms of chimeric polypeptide ligands that bind to specific receptors on cells, including growth factors belonging to angiopoietin family. Such polypeptide ligands are useful in promoting or inhibiting a differential function and/or influencing the phenotype, such as growth, survival, contractility, migration, and/or proliferation, of receptor-bearing cells such as endothelial cells, hematopoietic stem cells, and endothelial precursor cells. The invention also provides for nucleic acids encoding such polypeptide ligands, and both prokaryotic and eukaryotic expression systems for producing such polypeptide ligands. According to the invention, soluble forms of the polypeptide ligands described herein may be used to promote or inhibit biological responses in receptor-expressing cells.

The present invention is directed to a coiled coil chimeric molecule comprising a coiled-coil domain linked to a receptor binding domain of a ligand which forms a biologically active multimer, and wherein the chimeric molecule in its non-multimerio form is not biologically active.

Without limiting the source or structure of the coiled coil domain of the invention in any way, in one aspect, the coiled coil domain includes those belonging to a protein belonging to matrix protein family, transcription factor family, growth factor family or secretory protein family. Furthermore, the coiled coil domain may be of a matrix protein, in particular, cartilage matrix protein or cartilage oligomeric matrix protein.

In the coiled coil chimeric molecule, the receptor binding domain may bind to a variety of receptors, and in particular, Tie2 or Tie1 receptor. In one aspect, the receptor binding domain may be a fibrinogen-like domain of angiopoietin-1.

In another aspect of the invention, the coiled coil chimeric molecule may be linked directly or indirectly to an extracellular domain of the receptor.

The present invention is also directed to an isolated nucleic acid encoding a coiled coil chimeric molecule, which is discussed above. Further, the invention is directed to an expression vector that includes this nucleic acid. A host cell comprising the vector is also included within the purview of the invention.

The present invention also includes a soluble biologically active multimer comprising the coiled coil chimeric molecule discussed above. In particular, the multimer may be a homomer or a heteromer. If the multimer is a heteromer, then it is preferred that the coiled coil domain be heterogeneous with respect to at least one specie. The multimer may be a dimer, trimer, tetramer, pentamer, hexamer, hoptamer, octamer, nanomer or decamer. Moreover, the multimer may comprise coiled coil domains, which form parallel or anti-parallel structure.

The present invention is also directed to a method of promoting cell growth comprising contacting a coiled coil chimeric molecule comprising a coiled-coil domain linked to a receptor binding domain of a ligand as described above, to a population of cells that express receptors that are specific for the ligand, wherein said ligand is an agonist for said receptor, and wherein a multimeric form of the coiled coil chimeric molecule interacts with the receptor, which results in cell growth promotion. In particular, in this method, without being limited to any particular cell type, the cells may be endothelial cells, hematopoietic cells or other cells that express each specific receptor.

In another aspect of the invention, the present invention is directed to a method of promoting cell proliferation comprising contacting a coiled coil chimeric molecule comprising a coiled-coil domain linked to a receptor binding domain of a ligand as described above, to a population of cells that express receptors that are specific for the ligand, wherein said ligand is an agonist for said receptor, and wherein a multimeric form of the coiled coil chimeric molecule interacts with the receptor, which results in cell proliferation. In particular, in this method, without being limited to any particular cell type, the cells may be endothelial cells, hematopoietic cells or other cells that express each specific receptor.

In yet another aspect of the invention, the present invention is directed to a method of decreasing or inhibiting cell proliferation comprising contacting a coiled coil chimeric molecule comprising a coiled-coil domain linked to a ligand binding domain of a receptor as described above, to a population of cells that express ligands that are specific for the receptor, wherein a multimeric form of the coiled coil chimeric molecule interacts with the ligand, which results in decrease or inhibition of cell proliferation. In particular, in this method, without being limited to any particular cell type, the cells may be endothelial cells, hematopoietic cells or other cells that express each specific receptor.

In yet another aspect of the invention, the present invention is directed to a method of decreasing or inhibiting ligand activity comprising contacting a coiled coil chimeric molecule comprising a coiled-coil domain linked to a ligand binding domain of a receptor as described above, to a sample comprising a ligand that is specific for the receptor, wherein a multimeric form of the coiled coil chimeric molecule binds the ligand, which results in a decrease or inhibition of ligand activity.

In still another aspect of the invention, the present invention is directed to a method of making a chimeric molecule comprising: (A) recombinantly combining a nucleic acid encoding a coiled-coil domain with a nucleic acid encoding either a receptor binding region of a ligand or a ligand binding region of a receptor to produce a chimeric gene construct; and (B) expressing the gene construct in a host cell to produce the chimeric molecule. These and other objects of the invention will be more fully understood from the allowing description of the invention, the referenced drawings attached hereto and the claims appended hereto.

In yet another aspect of the invention, the invention is directed to a use of the invention molecule for the manufacture of a medicament for protecting endothelial cells against radiation damage to a mammal that has been exposed to irradiation, comprising administering a therapeutically effective amount of the coiled coil chimeric molecule described above to the mammal in need thereof.

### BRIEF DESCRIPTION OF THE DRAWINGS

The present invention will become more fully understood from the detailed description given herein below, and the accompanying drawings which are given by way of illustration only, and thus are not limitative of the present invention. It is understood that as used in the present application, cartilage matrix protein, CMP, matrilin-1, and MAT are used interchangeably.

FIGURE 1 shows a schematic structure of angiopoietin-1. Amino acids 1-19 are the secretory signal sequence (S), amino acids 158-255 are the coiled-coil oligomeric domain (CCOD), amino acids 226-283 are the linker (L), and amino acids 284-498 are the fibrinogen-like domain (FLD). There are cysteines (C) at amino acids 41, 54, 265, 315, 435, 437, 439, and 452.

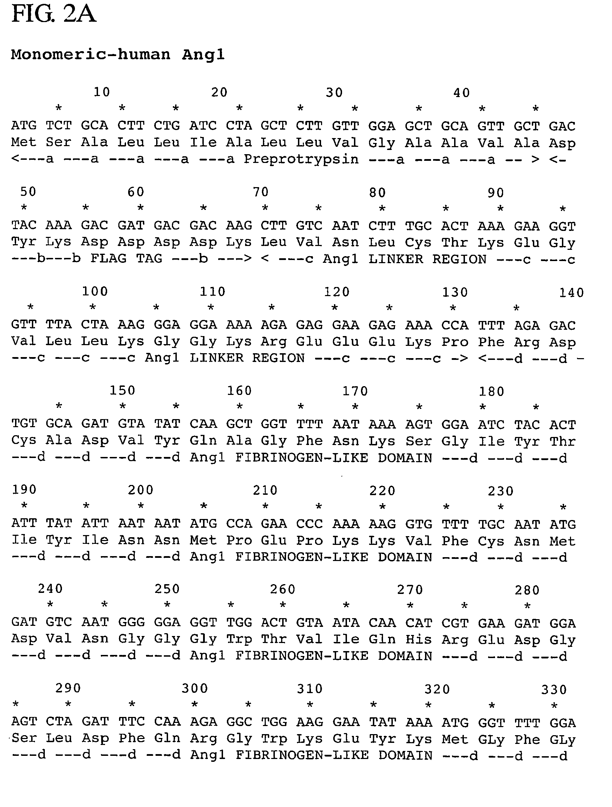

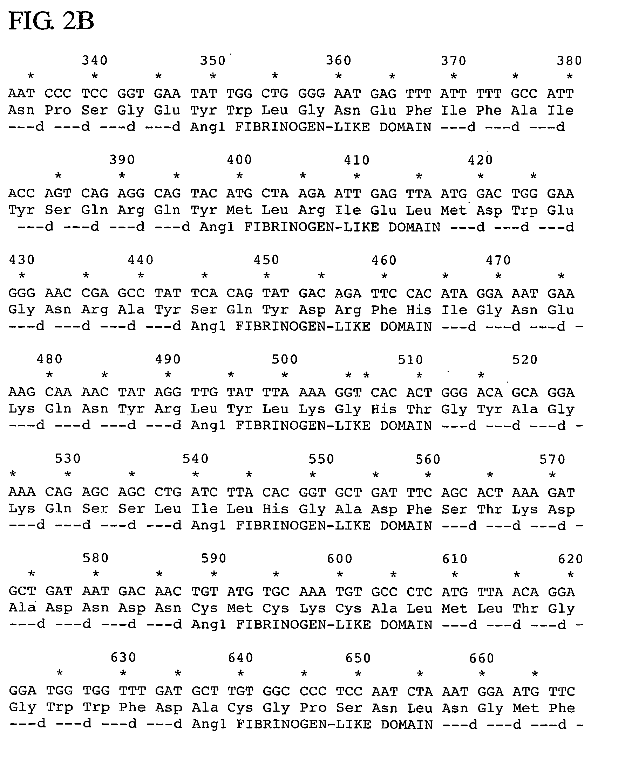

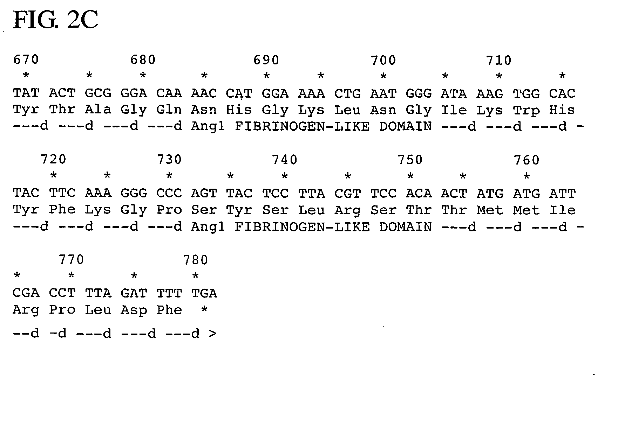

FIGURES 2A-2C show nucleic acid sequence (SEQ ID NO:1) and deduced amino acid sequence (SEQ ID NO:2) of Ang1/FD (fibrinogen-like domain of Ang1).

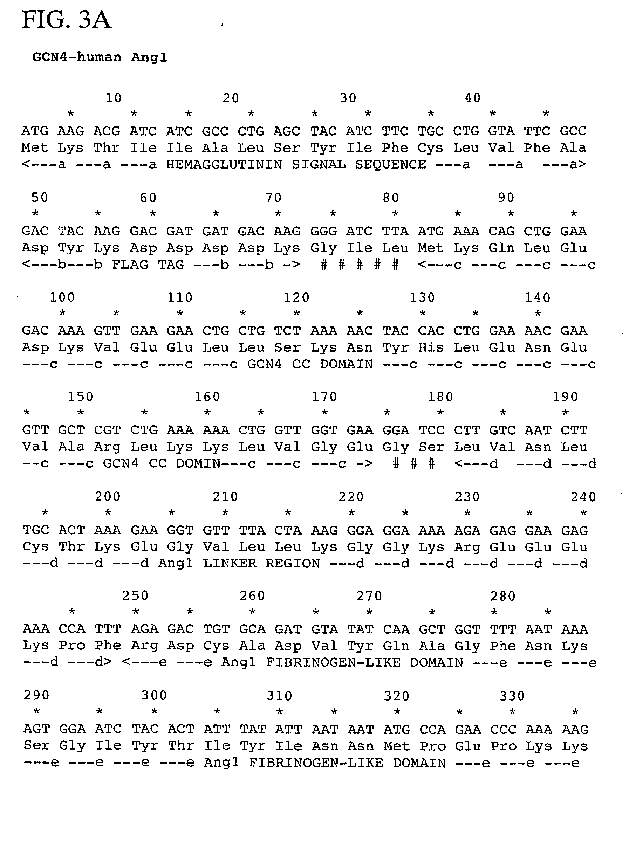

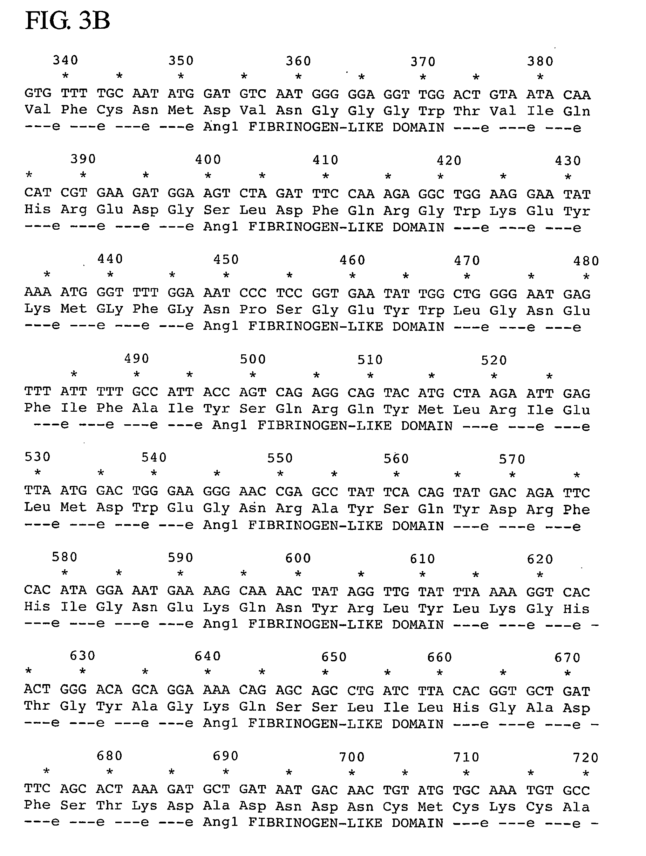

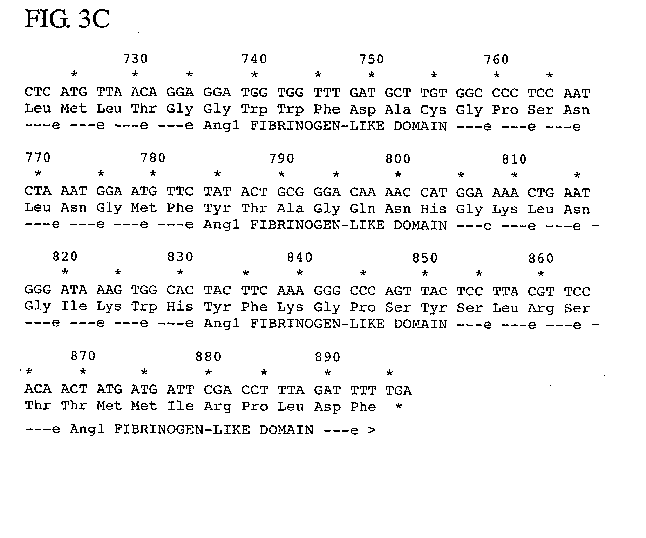

FIGURES 3A-3C show nucleic acid sequence (SEQ ID NO:3) and deduced amino acid sequence (SEQ ID NO:4) of GCN4/CC-Angl/FD (coiled-coil domain of GCN4-Ang1/FD).

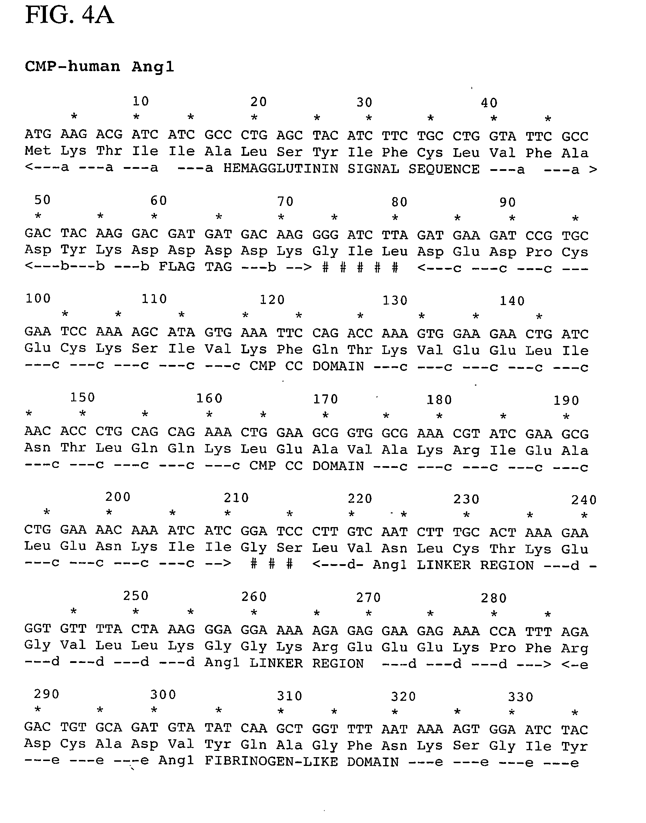

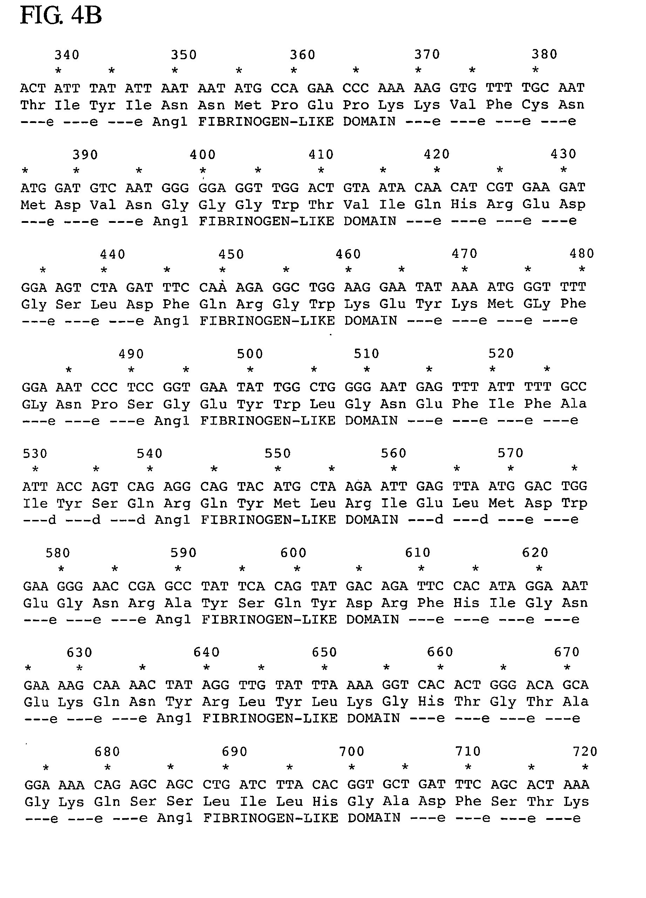

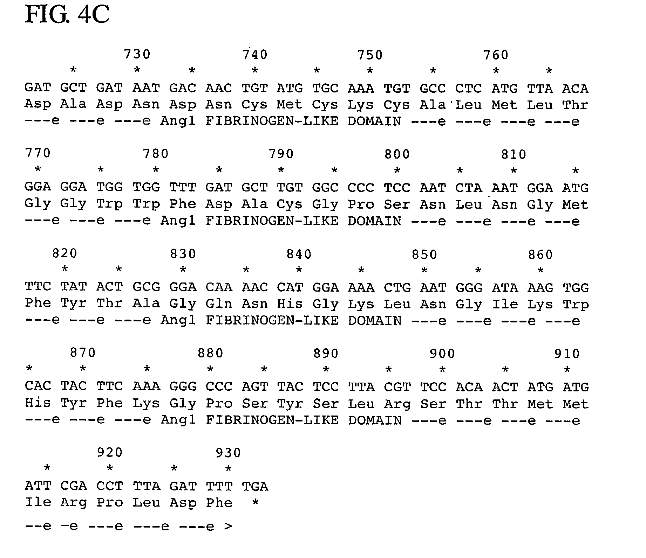

FIGURES 4A-4C show nucleic acid sequence (SEQ ID NO:5) and deduced amino acid sequence (SEQ ID NO:6) of CMP/CC-Angl/FD (coiled-coil domain of CMP-Ang1/FD).

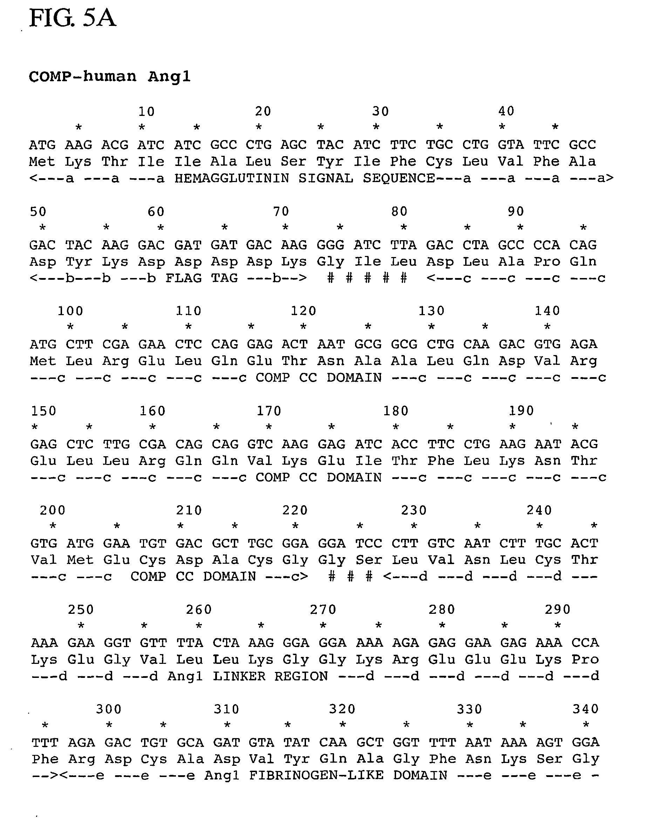

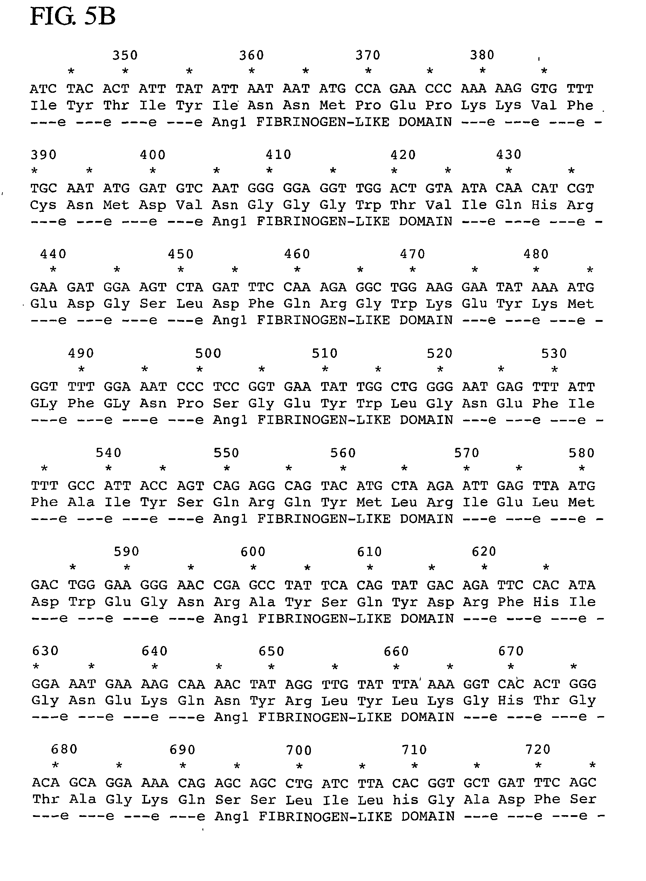

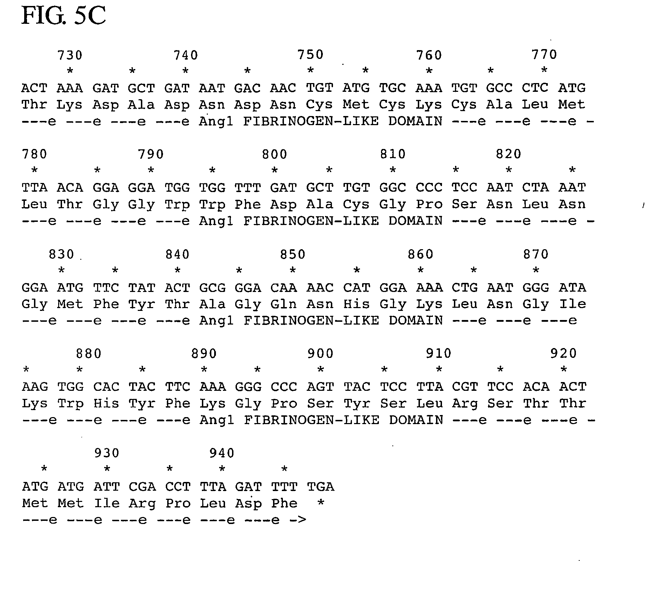

FIGURES 5A-5C show nucleic acid sequence (SEQ ID NO:7) and deduced amino acid sequence (SEQ ID NO:8) of COMP/CC-Angl/FD (coiled-coil domain of COMP-Ang1/FD).

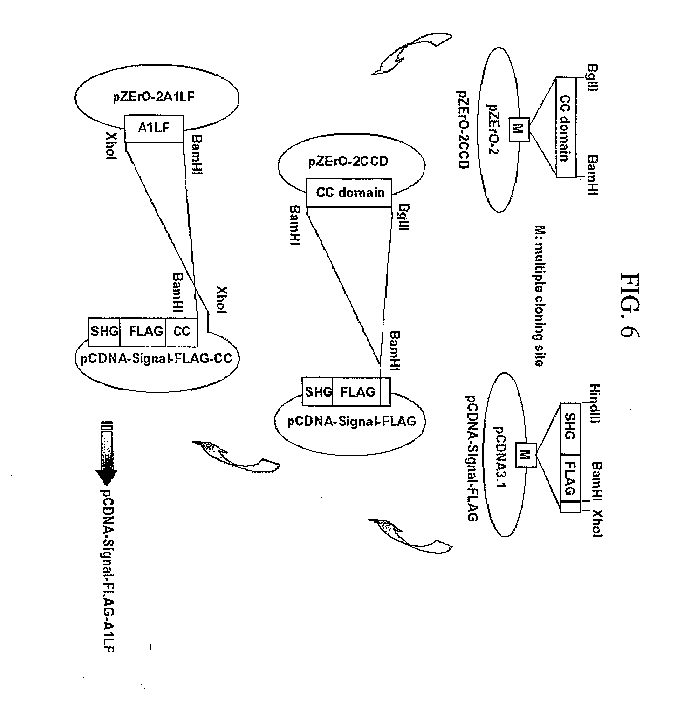

FIGURE 6 shows a schematic diagram for generation of gene constructs for multimeric chimeric-Ang1. "M" stands for multiple cloning site.

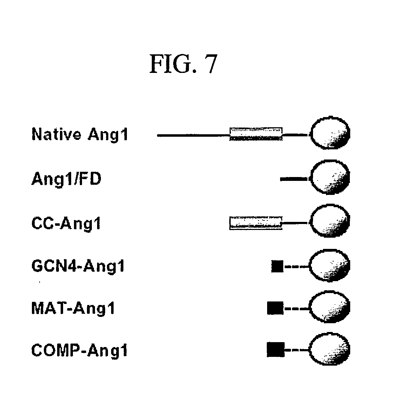

FIGURE 7 shows a schematic diagram of native Ang1, GCN4-Angl/FD, MAT-Ang1/FD, and COMP-Ang1/FD.

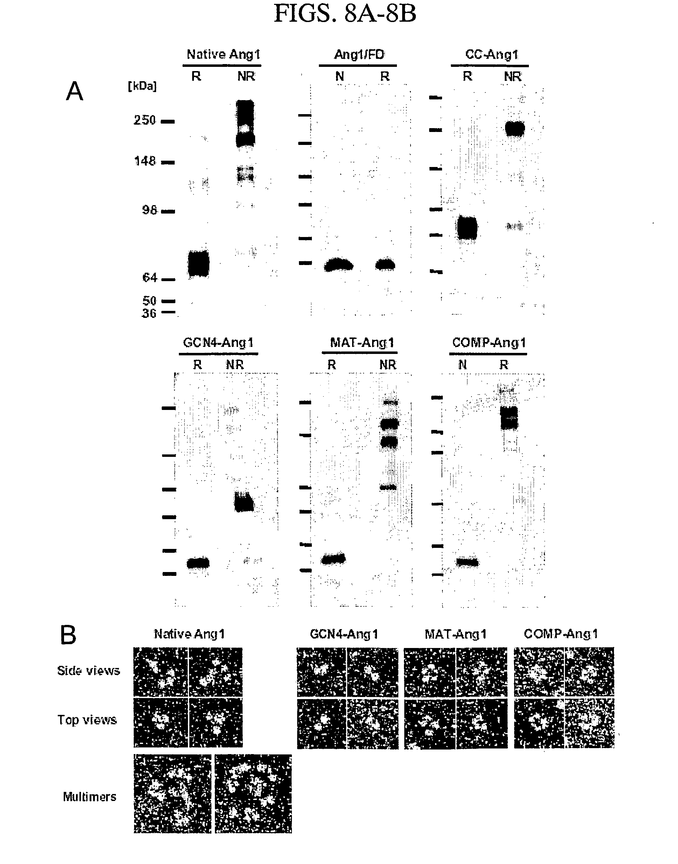

FIGURES 8A-8B show that Ang1 variants produce lower-order oligomers. FIGURE 8A shows molecular weight analysis of recombinant native Ang1, Ang1/FD, CC-Ang1, GCN4-Angl MAT/CC-Ang1, and COMP/CC-Ang1 under reducing (R) and non-reducing (NR) conditions. Numbers and bars indicate molecular sizes (kDa). 100 ng of each recombinant protein (reduced and non-reduced) is separated by 4-15% sodium dodecyl sulfate-polyacrylamide gel electrophoresis (SDS-PAGE), and electro-blotted to nitrocellulose membranes. The nitrocellulose membranes were Western blotted with anti-FLAG M1 antibody, washed, and incubated with horseradish peroxidase-conjugated secondary antibody. Signals were visualized by chemiluminescent detection according to the manufacturer's protocol (Amersham Pharmacia Biotech) and chemiluminescence scanner (LAS-1000, Fuji Film, Tokyo). FIGURE 8B shows glycerol sprayed/low-angle rotary metal-shadowed specimens that were imaged by TEM. Native Ang1 reveals variable-sized multimers of the basic 'mushroom-shaped' trimeric structure. CC-Angl reveals trimers, GCN4-Ang1 reveals dimers, MAT-Ang1 reveals mostly tetramer, and COMP-Ang1 reveals mostly pentamers.

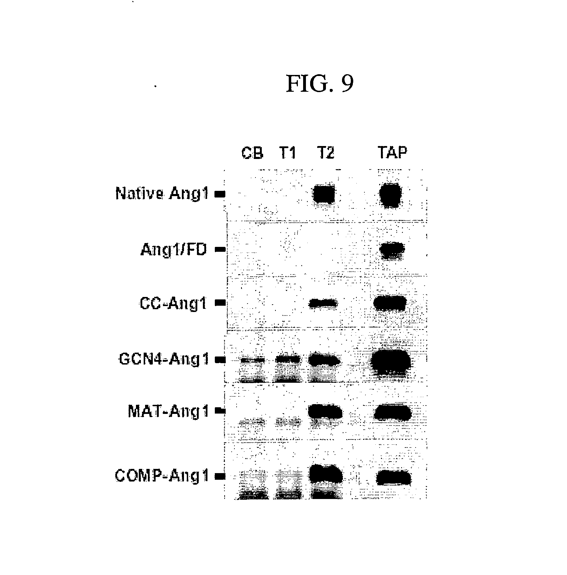

FIGURE 9 shows *in vitro* binding assay between recombinant native Ang1, Ang1/FD, CC-Ang1, GCN4-Angl MAT/CC-Ang1, and COMP/CC-Angl and soluble Tie1-Fc (sTie1-Fc, T1) and soluble Tie2-Fc (sTie2-Fc, T2). Approximately 75%, 0%, 8%, 20%, 95%, and 100% of the total protein amount of native Ang1, Ang1/FD, CC-Ang1, GCN4-Ang1, MAT-Ang1, and COMP-Angl, respectively, bound to Tie2. TAP, total amount of protein input for binding assay. Twenty nanograms of each protein was incubated with 100 ng of sTie1-Fc or sTie2-Fc for 4 hr, and then, protein-A conjugated agarose beads were added. The precipitate was separated by 10% SDS-PAGE, and electro-blotted on to nitrocellulose membranes. The nitrocellulose membranes were Western blotted with anti-FLAG M1 antibody, washed, and incubated with horseradish peroxidase-conjugated secondary antibody. Signals were visualized by chemiluminescent detection according to the manufacturer's protocol (Amersham Pharmacia Biotech) and chemiluminescence scanner (LAS-1000, Fuji Film, Tokyo).

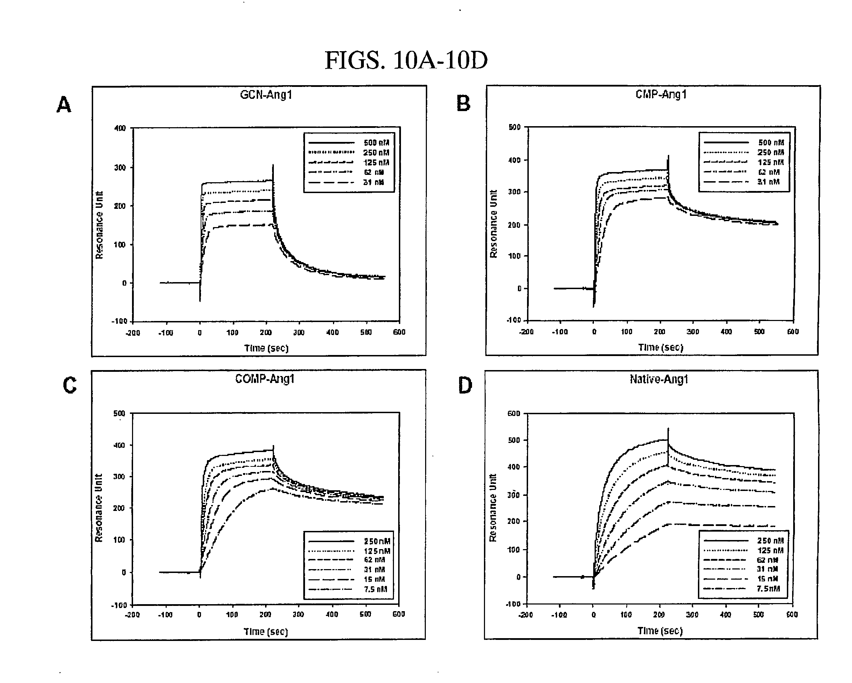

FIGURES 10A - 10D show *in vitro* binding assay between recombinant native Ang1, GCN4-Ang1/FD, MAT-Angl/FD, or COMP-Ang1/FD and immobilized soluble Tie2-Fc using Biacore assay. Each recombinant protein was passed over a Biacore sensor chip that had extracellular domain of Tie2-Fc and Fc protein covalently coupled to it, followed by dissembling in absence of the recombinant proteins. Concentrations of the recombinant proteins are shown in each panel. Specificity of binding was assessed by substracting Fc protein binding value from Tie2-Fc binding value during the measurement. Binding of the sensor chip is provided in resonance units (RUs). Maximal binding of each recombinant protein was observed at different concentrations. Binding of A, GCN4-Angl/FD and B, MAT-Ang1/FD were saturated at about 30 nM, while C, COMP-Ang1/FD, and D, native Ang1 binding saturation point was reached at over 125 nM. The binding affinity of GCN4-Ang1/FD, MAT-Ang1/FD, COMP-Angl/FD, and native Ang1 were estimated at 158.5 nM, 67 nM, 20.5 nM, and 7.5 nM (KD, dissociation constant), respectively.

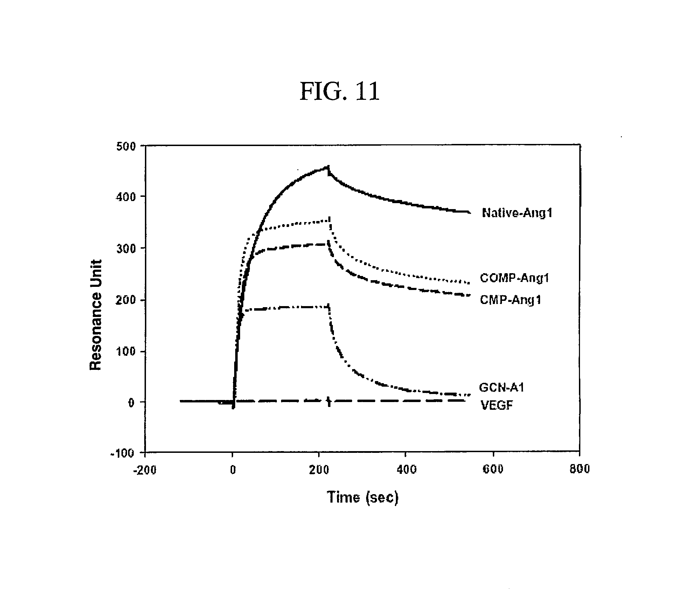

FIGURE 11 shows BIAcore analysis of native Ang1 (NA), GCN4-Ang1 (G), MAT-Ang1 (M) or COMP-Ang1 (C) with sTie2-Fc immobilized on a chip. Typical sensogram showing the association and dissociation profile of 60 nM of recombinant protein. An equal amount of VEGF was used as a control.

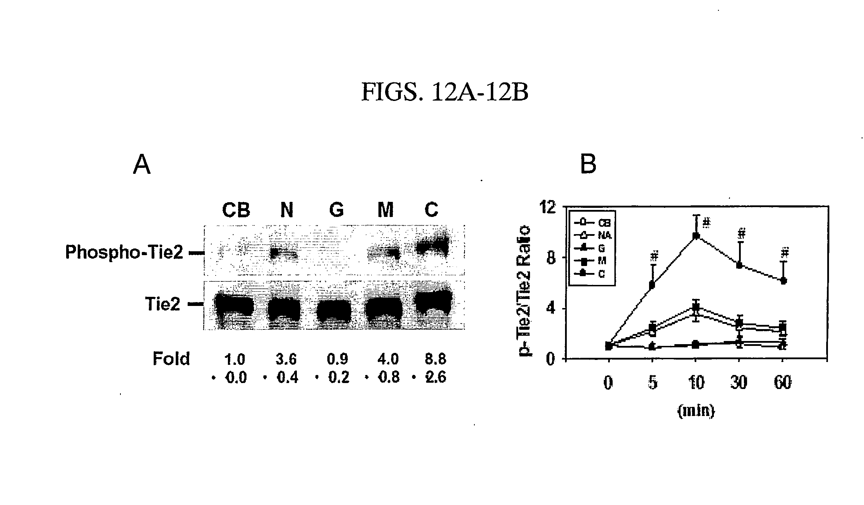

FIGURES 12A-12B show comparison of native Ang1 (N), GCN4-Ang1 (G), MAT-Ang1 (M) or COMP-Ang1 (C) in a Tie2 phosphorylation assay using human umbilical venous endothelial cells (HUVECs). Serum-starved HUVECs were treated with 200 ng/ml of native Ang1 or 100 ng/ml of Ang1 variants for 10 min (A), or the indicated times (B) and the phosphorylations of Tie2 were measured. Fold: Densitometric analyses are presented as the relative ratio of phospho-Tie2 to Tie2. The relative ratio of phospho-Tie2 (pTie2) to Tie2 at time 0 is arbitrarily presented as 1. Numbers represent the mean ± S.D. from 3 experiments. *, P<0.05 versus CB. #, P<0.05 versus NA. The cells were harvested in extraction buffer, and 0.5 mg of protein was used for immunoprecipitation. Tie2 proteins in the samples were immunoprecipitated with anti-Tie2 antibody and collected. Immunoprecipitated samples were Western blotted with anti-phospho-tyrosine antibody (upper panel), and the membrane was re-blotted with anti-Tie2 antibody to verify equal loading of protein in each lane (lower panel). Results were similar in three independent experiments.

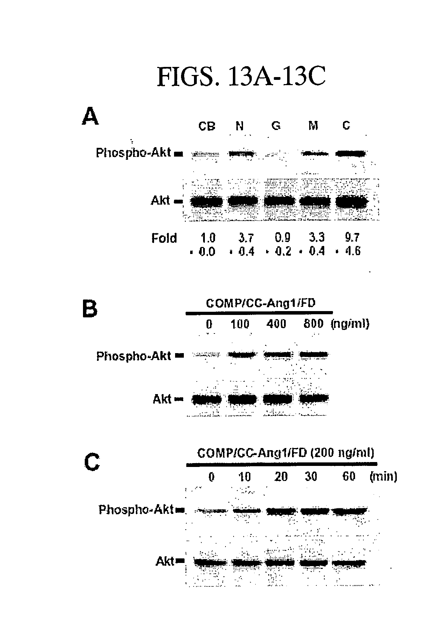

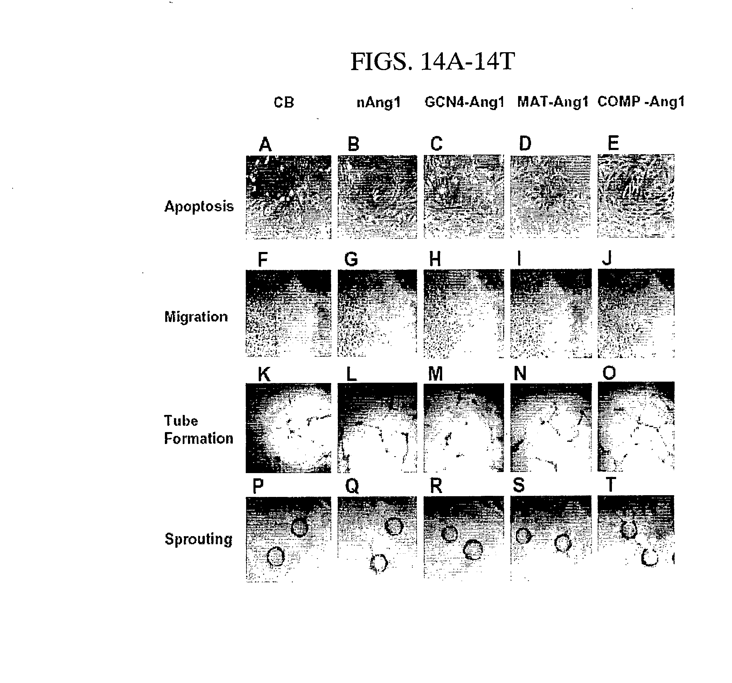

FIGURES 13A-13C show comparison of native Ang1, GCN4-Angl/FD, MAT-Angl/FD, or COMP-Ang1/FD in Akt (Ser473) phosphorylation assay in HUVECs. In each panel, upper band indicates that the Western blots were probed with anti-phospho-Akt (Ser473) antibody. Lower band shows the blot that was reprobed with anti-Akt antibody to verify equal loading of protein in each lane. **A,** HUVECs were incubated with control buffer (CB), and 200 ng of native Ang1 (N), GCN4-Angl/FD (G), MAT-Ang1/FD (M), or COMP-Ang1/FD (C) for 15 min. **B,** HUVECs were incubated with control buffer (CB) and indicated amount of COMP-Ang1/FD. **C**, HUVECs were incubated with 200 ng of COMP-Ang1/FD for indicated times. After treatment, cell lysates were harvested. Each lane contains 50 µg of total protein from the cell lysates. Results were similar in three independent experiments. Fold: Densitometric analyses are presented as the relative ratio of phospho-Akt (Ser472) to Akt. The relative ratio measured for control buffer was arbitrarily set at 1. Numbers represent the mean ± S.D. from three experiments. FIGURES 14A-14T show a comparison between native Ang1, GCN4-Angl, MAT-Ang1, and COMP-Ang1 in survival, migration, tube formation and sprouting activities of primary cultured endothelial cells. Representative photographs are shown. Native Ang1 and MAT-Ang1-induced notable increase in survival, migration, tube formation and sprouting activities. Notably, COMP-Ang1-induced survival, migration, tube formation and sprouting activities were greater than native Ang1- and MAT-Ang1- induced survival, migration, tube formation and sprouting activities. However, GCN4-Ang1 did not appear to exhibit any notable change in survival, migration, tube formation or sprouting activities.

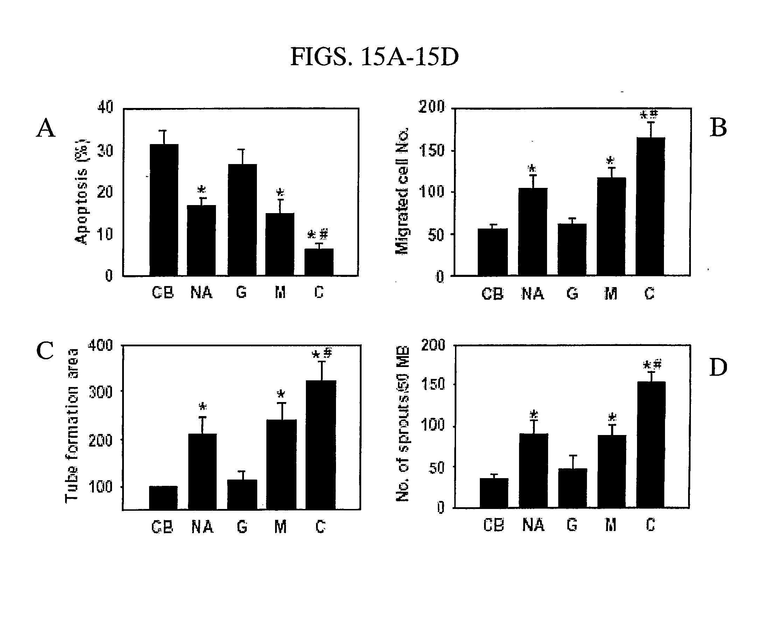

FIGURES 15A-15D show quantification of biological activities. CB, control buffer; NA, native Ang1; G, GCN4-Angl; M, MAT-Ang1; C, COMP-Ang1. *, P<0.05 versus CB. #, P<0.05 versus NA (200 ng/ml).

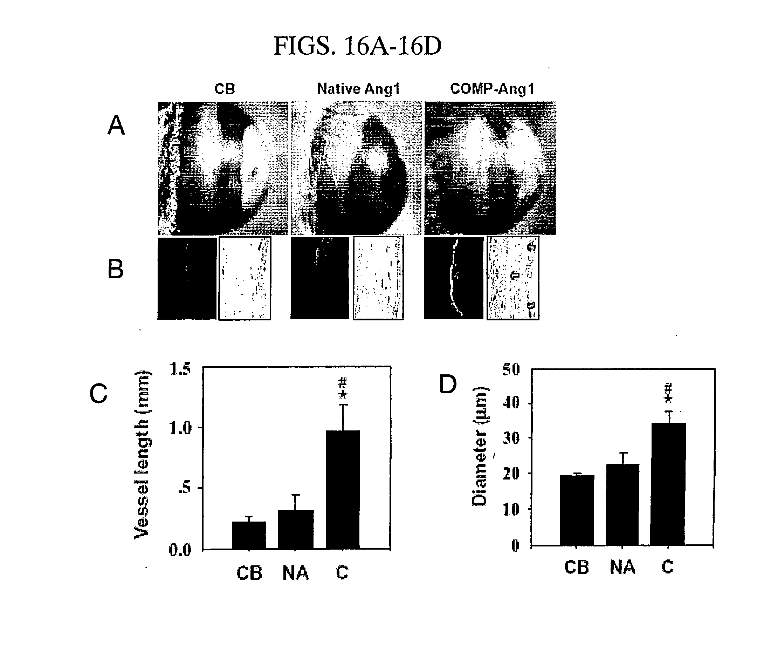

FIGURES 16A-16D show that COMP-Ang1 alone produces angiogenesis in the mouse corneal micropocket assay. **A**, Macroscopic photographs of mouse corneas. Corneal angiogenesis was not observed with control buffer (CB) or with native Ang1. COMP-Ang1 induced corneal angiogenesis extending from the limbus across the cornea. **B, left panels** show *in situ* BS-1 lectin fluorescent staining of corneal limbus vessels 6 days after pellet implantation. COMP-Ang1 increased the diameter of the limbar artery, while CB and native Ang1 induced no changes. **B, right panels** show hematoxylin and eosin staining confirms that COMP-Ang1 induced angiogenesis with significant vessel formation. Arrows indicate blood vessels containing red blood cells. **C-D** show vessel lengths (**C**) and diameters (**D**) of the limbar artery following the micropocket assay. *, *P*<0.01 *versus* CB (n=12). ^{#}, *P*<0.05 *versus* native Ang1 (n=12). COMP-Ang1 (n=12).

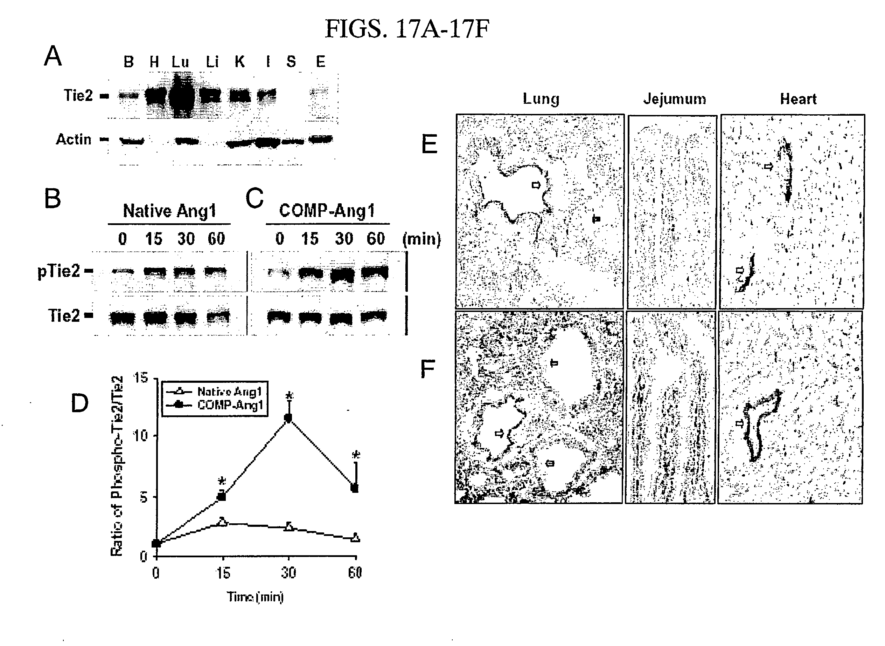

FIGURES 17A-17F show acute injection of COMP-Ang1 induces Tie2 phosphorylation in lung. **A** shows distribution of Tie2 in several organs including brain (B), heart (H), lung (Lu), liver (Li), kidney (K), small intestine (I), spleen (S), and ear (E) of adult mice. Protein lysates from each organ were immunoprecipitated and immunoblotted with anti-Tie2 antibody (upper panel). Each protein lysate was also immunoblotted with anti-actin antibody to verify equal amounts of total protein loaded (lower panel). **B-D** show *in vivo* phosphorylation of Tie2 stimulated by i.v. injection of native Ang1 (60 µg) or COMP-Ang1 (30 µg) assayed at the indicated times after injection. Tie2 was immunoprecipitated from lung protein lysate and immunoblotted with anti-phosphotyrosine to detect phosphorylated Tie2 (**B**, **C**-pTie2, upper panels). The membrane was stripped and reprobed with anti-Tie2 antibody (lower panels) to verify equal loading of protein in each lane. **D**, the relative ratio measured at time 0 is arbitrarily presented as 1. Dots represent the mean ± S.D. from 5 experiments. *, *P*<0.05 *versus* native Ang1. **E-F** show immunohistochemical staining of Tie2 counterstained with methyl green (**E**) and PECAM-1 counterstained with Meyer's hematoxylin (**F**). Most Tie2 staining corresponds to PECAM staining. White arrows indicate large blood vessels with positive staining. Gray arrows indicate bronchioles with negative staining.

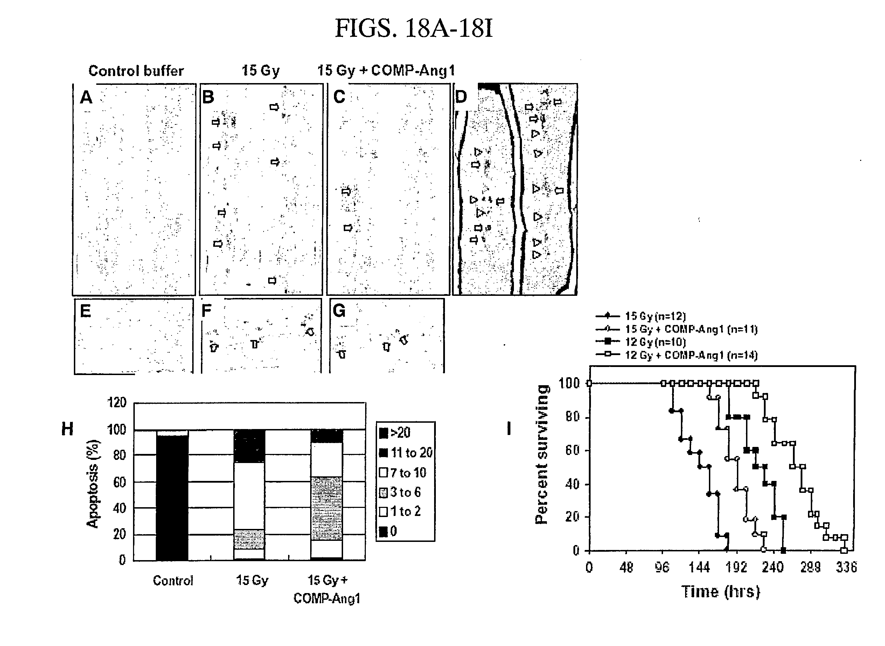

FIGURES 18A-18I show that COMP-Ang1 protects against radiation-induced microvascular apoptosis and death. **A-G** show mice which were given whole-body irradiation with 15 Gy and sacrificed after 4 hrs. While small intestinal villi and crypts from control mice have almost no apoptosis (**A, E**), villi and crypts from irradiated mice reveal extensive apoptosis (arrows) in lamina propria cells (**B**, **F**). I.V. injection of COMP-Ang1 markedly reduced radiation-induced apoptosis in lamina propria cells but not in cells of crypts (**C, G**). Co-staining of PECAM-1 (gray-pastel colors; arrow heads) and TUNEL (dark brown; arrows) allows for counting the number of apoptotic endothelial cells among the lamina propria cells (**D**). **H** shows frequency histograms of percent of apoptotic endothelial cells in the lamina propria of approximately 1,000 villi from each group. Small intestinal specimens were obtained 4 hrs after 15 Gy irradiation with or without i.v. injection of COMP-Ang1. Data represent mean scores from 5 experiments. **I** shows treatment with COMP-Ang1 prolongs survival in mice irradiated with 12 or 15 Gy. Survival was monitored every 12 hr after irradiation. Numbers in parentheses indicate animals per group. Two-tailed Fisher's exact test performed to compare control and COMP-Ang1-treated mice (*P* = 0.002 for 12 Gy; *P* = 0.003 for 15 Gy).

### DETAILED DESCRIPTION OF THE INVENTION

In the present application, "a" and "an" are used to refer to both single and a plurality of objects.

As described in greater detail below, applicants have discovered a method of using coiled-coil domains for "multimerizing" ligands, which enhances the biological activity of such ligands that, absent such multimerization, would have lower levels of biological activity. This method may be used to multimerize receptor binding domains from any ligand that has improved affinity and/or increased activity (i.e. signaling ability) when they were multimerized as compared to the non-multimerized form of the ligand.

The present invention also provides for methods of using coiled-coil domains for "multimerizing" soluble receptors, which functions to make otherwise inactive soluble receptors biologically active, or which enhances the biological and binding activity of receptors that, absent such multimerization, would have lower levels of biological and binding activity. This method may be used to multimerize ligand binding domains using any receptor, which has improved affinity and/or increased activity (i.e. binding) when they were multimerized as compared to the native form of the soluble receptor.

As used herein, "about" or "substantially" generally provides a leeway from being limited to an exact number. For example, as used in the context of the length of a polypeptide sequence, "about" or "substantially" indicates that the polypeptide is not to be limited to the recited number of amino acids. A few amino acids add to or subtracted from the N-terminus or C-terminus may be included so long as the functional activity such as its binding activity is present.

As used herein administration "in combination with" one or more further therapeutic agents includes simultaneous (concurrent) and consecutive administration in any order.

As used herein, "agonist" refers to a ligand that binds to a receptor, which activates the receptor and stimulates physiologic activity. For instance, Ang1 is considered to be an agonist of Tie2 receptor.

As used herein, "amino acid" and "amino acids" refer to all naturally occurring L-α-amino acids. This definition is meant to include norleucine, ornithine, and homocysteine.

As used herein, in general, the term "amino acid sequence variant" refers to molecules with some differences in their amino acid sequences as compared to a reference (e.g. native sequence) polypeptide. The amino acid alterations may be substitutions, insertions, deletions or any desired combinations of such changes in a native amino acid sequence.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Also included within the scope of the invention are proteins or fragments or derivatives thereof which exhibit the same or similar biological activity and derivatives which are differentially modified during or after translation, e.g., by glycosylation, proteolytic cleavage, linkage to an antibody molecule or other cellular ligand, and so on.

Insertional variants are those with one or more amino acids inserted immediately adjacent to an amino acid at a particular position in a native amino acid sequence. Immediately adjacent to an amino acid means connected to either the α-carboxy or α-amino functional group of the amino acid.

Deletional variants are those with one or more amino acids in the native amino acid sequence removed. Ordinarily, deletional variants will have one or two amino acids deleted in a particular region of the molecule.

As used herein, "antagonist" refers to a ligand that tends to nullify the action of another ligand, as a ligand that binds to a cell receptor without eliciting a biological response.

As used herein, "biologically active" with regard to the ligand of the present invention refers to the ability of a molecule to specifically bind to and signal through a native receptor, e.g., a native Tie2 receptor, or to block the ability of a native Tie receptor (e.g., Tie2) to participate in signal transduction. Thus, the (native and variant) ligands of the present invention include agonists and antagonists of a native receptor, e.g. Tie2 receptor. Preferred biological activities of the ligands of the present invention include the ability to induce or inhibit vascularization. The ability to induce vascularization will be useful for the treatment of biological conditions and disease, where vascularization is desirable. On the other hand, the ability to inhibit or block vascularization may, for example, be useful in preventing or attenuating cell proliferation and tumor growth.

Preferred biological activities of the ligands of the present invention include the ability to inhibit vascular permeability. The ability to inhibit vascular permeability will be useful for treatment of medical conditions and diseases such as diabetic retinopathy, edema, and ascites. Preferred biological activities of the ligands of the present invention include the ability to maintain endothelial cell integrity (including preventing apoptosis). The ability to maintain endothelial cell integrity will be useful for treatment of medical conditions and diseases such as mannitol treatment, irradiation, and sepsis.

The biological activity of the chimeric receptor, which may be in soluble form, includes its ability to inhibit or competitively inhibit the ligand's activity by binding to its ligand. Thus, in this way, cell proliferation may be inhibited if the ligand is an agonist for cell proliferation. Alternatively, administration of chimeric receptor may act as an enhancer of cell proliferation if the ligand is an antagonist for cell proliferation.

It is also contemplated that chimeric ligand and chimeric receptor be labeled with a detectable label, such as radioisotope, fluorescent tag, enzymatic tag, or a chemiluminescent tag to determine ligand-receptor binding interaction. As such, assay systems employing the chimeric molecule is also contemplated.

As used herein, "carriers" include pharmaceutically acceptable carrier, excipients, or stabilizers which are nontoxic to the cell or mammal being exposed thereto at the dosages and concentrations employed. Often the pharmaceutically acceptable carrier is an aqueous pH buffered solution. Examples of pharmaceutically acceptable carriers include without limitation buffers such as phosphate, citrate, and other organic acids; antioxidants including ascorbic acid; low molecular weight (less than about 10 residues) polypeptide; proteins, such as serum albumin, gelatin, or immunoglobulins; hydroplilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{®}, polyethylene glycol (PEG), and PLURONICS^{®}.

As used herein, "chimeric ligand", "chimeric receptor", "chimeric polypeptide" or "chimeric molecule" refers to the combination of coiled coil domain and a receptor binding domain or a ligand binding domain. The resultant chimeric polypeptide is capable of forming biologically active multimers, which are soluble. The coiled coil domain may be derived from any source, including any animal or mammalian protein, and in particular any human protein, and further includes those that are synthetically made. Moreover, the coiled coil domain and the ligand or receptor constructs may be from the same or different source. It is understood that the chimeric construct comprises the coiled coil domain and a receptor binding domain of a ligand or a ligand binding domain of a receptor, and further may include other components that may be included so long their inclusion does not interfere with the formation of a biologically active multimer that has improved solubility, ease of recombinant production of the chimeric polypeptide and substantially similar or greater potency as the native ligand or native soluble receptor. For example, FLAG sequence may be included for ease of purification, provided its inclusion does not interfere with the function of the chimeric molecule. The FLAG sequence also may be removed if a humanized construct is desired.

As used herein, "effective amount" is an amount sufficient to effect beneficial or desired clinical or biochemical results. An effective amount can be administered one or more times. An effective amount of an inhibitor compound is an amount that is sufficient to palliate, ameliorate, stabilize, reverse, allow or delay the progression of the disease state.

As used herein, "fragments" or "functional derivatives" refers to biologically active amino acid sequence variants and fragments of the native ligands or receptors of the present invention, as well as covalent modifications, including derivatives obtained by reaction with organic derivatizing agents, post-translational modifications, derivatives with nonproteinaceous polymers, and immunoadhesins.

As used herein, "host cell" includes an individual cell or cell culture which can be or has been a recipient of a vector of this invention. Host cells include progeny of a single host cell, and the progeny may not necessarily be completely identical (in morphology or in total DNA complement) to the original parent cell due to natural, accidental, or deliberate mutation and/or change.

As used herein, "ligand" refers to any molecule or agent, or compound that specifically binds covalently or transiently to a molecule such as a polypeptide. When used in certain context, ligand may include antibody. In other context, "ligand" may refer to a molecule sought to be bound by another molecule with high affinity, such as in a ligand trap.

As used herein, "ligand binding domain" refers to the portion of the receptor that binds to the ligands and includes the minimal portion of the receptor that is necessary to bind its ligand.

As used herein, "linked" refers to direct or indirect connection between the multimerizing domain and the ligand or receptor. Both a direct fusion between these two domains or indirect fusion as by the domains being separated by a linker or an intervening domain or element are contemplated, so long as the activity of the chimeric fusion is present.

As used herein, "mammal" for purposes of treatment refers to any animal classified as a mammal, including humans, domestic and farm animals, and zoo, sports, or pet animals, such as dogs, cats, cattle, horses, sheep, pigs, and so on. Preferably, the mammal is human.

As used herein, "multimer" or "multimeric" refers to the joining of the multimerizing agent such as the coiled coil domain to each other to form a dimer, trimer, tetramer, pentamer, hexamer, heptamer, octamer, nanomer, decamer and so on, which may be in a parallel or anti-parallel form, through intramolecular or intermolecular bonds.

As used herein "pharmaceutically acceptable carrier and/or diluent" includes any and all solvents, dispersion media, coatings antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired.

The principal active ingredient is compounded for convenient and effective administration in effective amounts with a suitable pharmaceutically acceptable carrier in dosage unit form. A unit dosage form can, for example, contain the principal active compound in amounts ranging from 0.5 µg to about 2000 mg. Expressed in proportions, the active compound is generally present in from about 0.5 µg/ml of carrier. In the case of compositions containing supplementary active ingredients, the dosages are determined by reference to the usual dose and manner of administration of the said ingredients.

As used herein, "receptor binding domain" refers to the portion of the ligand that binds to the receptor and includes the minimal portion of the ligand that is necessary to bind its receptor. The present invention is based on the discovery that a multimerizing agent, such as a coiled coil domain, which was previously perceived as a source of hindrance for isolating recombinant proteins containing them, has been found to provide advantageous features of easy recombinant protein expression and purification, greater solubility and greater or substantially equal potency compared with the native protein containing the coiled coil domain.

As used herein, "sample" or "biological sample" is referred to in its broadest sense, and includes any biological sample obtained from an individual, body fluid, cell line, tissue culture, or other source which may contain a chimeric Angl binding factor, depending on the type of assay that is to be performed. As indicated, biological samples include body fluids, such as semen, lymph, sera, plasma, urine, synovial fluid, spinal fluid and so on. Methods for obtaining tissue biopsies and body fluids from mammals are well known in the art.

As used herein, the term "specifically binds" refers to a non-random binding reaction between two molecules, for example between an antibody molecule immunoreaoting with an antigen, or a non-antibody ligand reacting with another polypeptide, such as chimeric Ang1 specifically binding with Tie2.

As used herein, "subject" is a vertebrate, preferably a mammal, more preferably a human.

As used herein, "treatment" is an approach for obtaining beneficial or desired clinical results. Beneficial or desired clinical results include, but are not limited to, alleviation of symptoms, diminishment of extent of disease, stabilized (i.c., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. "Treatment" refers to both therapeutic treatment and prophylactic or preventative measures. Those in need of treatment include those already with the disorder as well as those in which the disorder is to be prevented. "Palliating" a disease means that the extent and/or undesirable clinical manifestations of a disease state are lessened and/or the time course of the progression is slowed or lengthened, as compared to a situation without treatment.

As used herein, "vector,", "polynucleotide vector", "construct" and "polynucleotide construct" are used interchangeably herein. A polynucleotide vector of this invention may be in any of several forms, including, but not limited to, RNA, DNA, RNA encapsulated in a retroviral coat, DNA encapsulated in an adenovirus coat, DNA packaged in another viral or viral-like form (such as herpes simplex, and adeno-associated virus (AAV)), DNA encapsulated in liposomes, DNA complexed with polylysine, complexed with synthetic polycationic molecules, complexed with compounds such as polyethylene glycol (PEG) to immunologically "mask" the molecule and/or increase half-life, or conjugated to a non-viral protein. Preferably, the polynucleotide is DNA. As used herein, "DNA" includes not only bases A, T, C, and G, but also includes any of their analogs or modified forms of these bases, such as methylated nucleotides, internucleotide modifications such as uncharged linkages and thioates, use of sugar analogs, and modified and/or alternative backbone structures, such as polyamides.

The present invention includes a multimers forming domain. In particular, coiled coil domain is exemplified. The coiled coil domain may be any amino acid sequence that forms a coiled coil structure. While the exemplified coiled coil domains herein are those cloned from a variety of proteins, it is understood that various mutations and derivatization are encompassed by the invention, so long as the resultant coiled coil domain is recognized by a person of skill in the art as a coiled coil structure and the coiled coil domain containing chimera is capable of forming a multimer, is easily soluble, and is able to provide similar or greater potency with respect to the native ligand or receptor.

It is further understood that in certain situations, in linking together the multimerzing domain with the receptor binding domain of the ligand the multimerizing domain and the binding domain may be from the same protein, or they may be from different proteins. For instance, Ang1 coiled coil domain may be linked to its own fibrinogen-like domain in a more efficiently manner. Or, a cartilage oligomeric matrix protein (COMP) could be linked to the Ang1 fibrinogen-like domain.

### Chimeric Ang1

When mention is made of the chimeric constructs GCN-Angl, MAT-Angl, or COMP-Angl, it is understood that the Ang1 portion referred to is the fibrinogen domain of Ang1. In addition, MAT-Ang1 is also sometimes referred to as CMP-Angl, CMP-Angl/FD, or CMP/CC-Angl/FD in the figures.

Applicant has discovered that a chimeric form of Angel, COMP-Angl, has many potential advantages over the native protein The generation of native Ang1 is difficult, and the activities of the purified proteins vary, possibly due to its tendency to form multimers. Our structural analysis of native Aug1 by rotary shadowing TEM indicates that native Ang1 exists as variably-sized multimers. Though we originally intended to generate trimeric and pentameric Angl using short coiled-coil domains of matrix proteins, interestingly, MAT-Angl and COMP-Ang1 yielded additional oligomers. Nevertheless, MAT-Ang1 and COMP-Angl are easier to purify and more soluble than native Ang1. Notably, COMP-Angl is approximately 3-5 times more potent than native Ang1. Without being limited by theory, there may be two possible reasons why COMP-Ang1 produced the most potent effect on Tie2 and Akt phosphorylation. First, COMP-Angl could be the most biologically active among the variants because of its rapid association and dissociation rate. Second, COMP-Angl may induce Tie2 clustering in endothelial caveolae more efficiently than the other recombinant Angl proteins. Our data indicate that Tie2 molecules are localized in endothelial caveolae. Thus, an engineered Ang1 protein with an oligomeric structure better suited to clustering Tie2 in caveolae may better facilitate Tie2 multimerization and phosphorylation.

In a mouse corneal micropocket assay, Ang1 failed to stimulate an angiogenic response when administered alone. However, when co-administered with VEGF, Ang1 augmented postnatal angiogenesis. We have shown that native Ang1 failed to stimulate an angiogenic response. However, COMP-Angl alone stimulates angiogenesis. Furthermore, COMP-Angl produced an increase in the luminal diameter of the basal limbus. This role is consistent with increased frequency of enlarged vessel diameters in Ang1-overexpressing transgenic mice (Suri, C. et al. 1998, Science 282: 468-471; Thurston, G. et al. 1999, Science 286:2511-2514). In a rabbit ischemic hindlimb model, we previously showed that Ang1 gene delivery resulted in larger blood vessels compared to VEGF gene delivery (Chae, J. K. et al. 2000, Arterioscler. Thromb. Vasc. Biol. 20:2573-2578). Thus, COMP-Angl can produce more effective blood flow by increasing the diameter of arterial lumens. In addition, Angl can counteract VEGF-induced side effects such as edema and inflammation, while having an additive effect on angiogenesis (Kwak, H. J. et al. 2000, Circulation 101:2317-2324; Thurston, G. et al. 1999, Science 286:2511-2514; Kim, I. et al. 2001, Circ. Res. 89:477-479). Thus, COMP-Angl protein delivery may be useful for accurate and safe therapeutic angiogenesis.

Integrity of the vascular endothelium in response to physical, biochemical, and immune-mediated damage is important to maintaining endothelial function and preventing vascular diseases (Cines, D.B. et al. 1998, Blood 91:3527-3561). According to a recent report, extensive apoptosis of microvascular endothelial cells of the lamina propria is the primary lesion initiating intestinal radiation damage (Paris, F. et al. 2001, Science 293:293-297). Thus, GI tract damage during abdominal radiotherapy limits the dose that can be used during cancer treatment. Radiation damage to vascular endothelial cells can be prevented by VEGF (Okunieff, P. et al. 1998, Radiat. Res. 150:204-211) or basic fibroblast growth factor (bFGF) (Paris, F. et al. 2001, Science 293:293-297). Importantly, VEGF receptors are expressed in endothelial cells that are actively involved in vasculogenesis and angiogenesis such as tumor progression, whereas bFGF receptors are expressed in both endothelial and non-endothelial cells including cancer cells (Paris, F. et al. 2001, Science 293:293-29; Veikkola, T. & Alitalo, K. 1999, Seminars in Cancer Biol. 9:211-220). Therefore, administration of VEGF and bFGF for protection of endothelial damage may help tumor progression. Interestingly, Tie2 is selectively expressed in active form in the endothelial cells of normal adult vessels (Wong, A.L. et al. 1997, Circ. Res. 81:567-574). We recently reported that the angiopoietin/Tie2 system in normal adult blood vessels may be important in maintaining the integrity of non-proliferating endothelial cells (Kim, I. et al. 2001, Cardiovas. Res. 49:872-881). Our immunohistochemical staining indicates that Tie2 is selectively expressed in most endothelial cells of normal adult vessels and capillaries. Moreover, Tie2 is effectively activated by acute administration of COMP-Angl. One concern is that repeated administration of Ang1 may increase tumor angiogenesis during radiation therapy. However, the role of Ang1 in tumor angiogenesis is still controversial. It has even been suggested that Angl may suppress tumor progression through 'stabilization' of tumor vessels (Tian, S. et al. 2002, Br. J. Cancer 286:645-651; Hawighorst, T. et al. 2002, Am. J. Pathol. 160:1381-1392). Therefore, using Ang1 for protection against radiation-induced endothelial cell damage could be ideal. Indeed, COMP-Angl treatment strongly protects against extensive radiation-induced extensive endothelial apoptosis in villi, but has no observed effect on non-endothelial cells. In addition, COMP-Angl treatment prolongs survival periods, perhaps as a result of decreasing damage to the GI tract. Optimizing the dosage and route of administration of COMP-Angl could further improve endothelial cell survival following radiation-induced endothelial cell damage.

COMP-Angl is superior to native Ang1 in several ways including efficiency of generation, potency, Tie2 activation *in vivo*, angiogenesis, and protection against endothelial injury *in vivo*. It seems likely that it can be further applied to the prevention of vascular leakage, protection against sepsis-induced endothelial cell injury, enhancement of re-endothelialization after angioplasty, and *in vitro* amplification of Tie2 positive endothelial precursor stem cells. In conclusion, we designed and generated a soluble, non-aggregating, potent, and stable chimeric Ang1 variant, COMP-Angl. It may be useful for clinical therapies including therapeutic angiogenesis and endothelial cell protection.

### Coiled Coil

The α-helical coiled coil is probably the most widespread subunit oligomerization motif found in proteins. Accordingly, coiled coils fulfill a variety of different functions. In several families of transcriptional activators, for example, short leucine zippers play an important role in positioning the DNA-binding regions on the DNA (Ellenberger et al., 1992, Cell 71:1223-1237). Coiled coils are also used to form oligomers of intermediate filament proteins. Coiled-coil proteins furthemore appear to play an important role in both vesicle and viral membrane fusion (Skehel and Wiley, 1998, Cell 95:871-874). In both cases hydrophobic sequences, embedded in the membranes to be fused, are located at the same end of the rod-shaped complex composed of a bundle of long α-helices. This molecular arrangement is believed to cause close membrane apposition as the complexes are assembled for membrane fusion.

The coiled coil is often used to control oligomerization. It is found in many types of proteins, including transcription factors such as, but not limited to GCN4, viral fusion peptides, SNARE complexes and certain tRNA synthetases, among others. Very long coiled coils are found in proteins such as tropomyosin, intermediate filaments and spindle-pole-body components.

Coiled coils involve a number of α-helices that are supercoiled around each other in a highly organized manner that associate in a parallel or an antiparallel orientation. Although dimers and trimers are the most common. The helices may be from the same or from different proteins.

The coiled-coil is formed by component helices coming together to bury their hydrophobic seams. As the hydrophobic seams twist around each helix, so the helices also twist to coil around each other, burying the hydrophobic seams and forming a supercoil. It is the characteristic interdigitation of side chains between neighbouring helices, known as knobs-into-holes packing, that defines the structure as a coiled coil. The helices do not have to run in the same direction for this type of interaction to occur, although parallel conformation is more common. Antiparallel conformation is very rare in trimers and unknown in pentamers, but more common in intramolecular dimers, where the two helices are often connected by a short loop.

In the extracellular space, the heterotrimeric coiled-coil protein laminin plays an important role in the formation of basement membranes. Other examples are the thrombospondins and cartilage oligomeric matrix protein (COMP) in which three (thrombospondins 1 and 2) or five (thrombospondins 3, 4 and COMP) chains are connected. The molecules have a flower bouquet-like appearance, and the reason for their oligomeric structure is probably the multivalent interaction of the C-terminal domains with cellular receptors.

**GCN4**

The yeast transcriptional activator GCN4 is 1 of over 30 identified eukaryotic proteins containing the basic region leucine zipper (bZIP) DNA-binding motif (Ellenberger et al., 1992, Cell 71:1223-1237). The bZIP dimer is a pair of continuous alpha helices that form a parallel coiled-coil over their carboxy-teminal 34 residues and gradually diverge toward their amino termini to pass through the major groove of the DNA binding site. The coiled-coil dimerization interface is oriented almost perpendicular to the DNA axis, giving the complex the appearance of the letter T. bZIP contains a 4-3 heptad repeat of hydrophobic and nonpolar residues that pack together in a parallel alpha-helical coiled-coil (Ellenberger et al., 1992, Cell 71:1223-1237). The stability of the dimer results from the side-by-side packing of leucines and nonpolar residues in positions a and d of the heptad repeat, as well as a limited number of intra- and interhelical salt bridges, shown in a crystal structure of the GCN4 leucine zipper peptide (Ellenberger et al., 1992,Cell 71:1223-1237).

**Cartilage Matrix Protein (CMP or MAT)**

CMP (matrilin-1) was isolated from bovine tracheal cartilage as a homotrimer of subunits of *M*ᵣ 52,000 (Paulsson and Heinegård, 1981, Biochem J. 197:367-375), where each subunit consists of a vWFA1 module, a single EGF domain, a vWFA2 module and a coiled coil domain spanning five heptads (Kiss et al., 1989, J. Biol. Chem. 264:8126-8134; Hauser and Paulsson, 1994, J. Biol. Chem. 269:25747-25753). Electron microscopy of purified CMP showed a bouquet-like trimer structure in which each subunit forms an ellipsoid emerging from a common point corresponding to the coiled coil (Hauser and Paulsson, 1994, J. Biol. Chem. 269:25747-25753). The coiled coil domain in matrilin-1 has been extensively studied. The trimeric structure is retained after complete reduction of interchain disulfide bonds under non-denaturing conditions (Hauser and Paulsson, 1994, J. Biol. Chem. 269:25747-25753).

**Cartilage Oligomeric Matrix Protein (COMP)**

A non-collagenous glycoprotein, COMP, was first identified in cartilage (Hedbom et al., 1992, J.Biol.Chem. 267:6132-6136). The protein is a 524 kDa homopentamer of five subunits which consists of an N-terminal heptad repeat region (cc) followed by four epidermal growth factor (EGF)-like domains (EF), seven calcium-binding domains (T3) and a C-terminal globular domain (TC). According to this domain organization, COMP belongs to the family of thrombospondins. Heptad repeats (*abcdefg*)" with preferentially hydrophobic residues at positions *a* and *d* form -helical coiled-coil domains (Cohen and Perry, 1994, Science 263:488-489). Recently, the recombinant five-stranded coiled-coil domain of COMP (COMPcc) was crystallized and its structure was solved at 0.2 nm resolution (Malashkevich et al.,1996, Science 274:761-765).

**Nucleic Acid Constructs**

The present invention also provides for a nucleic acid encoding a fusion polypeptide wherein the fusion polypeptide comprises a first subunit comprising at least one copy of the receptor binding domain of a ligand, the first subunit being fused to the C-terminal end of a multimerizing component.

Alternatively, the present invention provides for a nucleic acid encoding a fusion polypeptide wherein the fusion polypeptide comprises a first subunit comprising at least one copy of the receptor binding domain of a ligands, the first subunit being fused to the N-terminal end of a multimerizing component In particular, the multimerizing component may be the coiled coil domain.

Also provided is an expression vector comprising a nucloic acid molecule of the invention as described herein, wherein the nucleic acid molecule is operatively linked to an expression control sequence. Also provided is a host-vector system for the production of a fusion polypeptide which comprises the expression vector of the invention which has been introduced into a host cell suitable for expression of the fusion polypeptide. The suitable host cell may be a bacterial cell such as *E*. *coli*, a yeast cell, such as *Pichia pastoris*, an insect cell, such as *Spodoptera frugiperda*, or a mammalian cell, such as a COS or CHO cell.

The present invention also contemplates methods of producing the fusion polypeptides of the invention by growing cells of the host-vector system described herein, under conditions permitting production of the fusion polypeptide and recovering the fusion polypeptide so produced. The fusion polypeptides useful for practicing the present invention may be prepared by expression in a prokaryotic or eukaryotic expression system.

The recombinant gene may be expressed and the polypeptide purified utilizing any number of methods. The gene may be subcloned into a bacterial expression vector, such as for example, but not by way of limitation, pZErO.

The fusion polypeptides may be purified by any technique which allows for the subsequent formation of a stable, biologically active protein. For example, and not by way of limitation, the factors may be recovered from cells either as soluble proteins or as inclusion bodies, from which they may be extracted quantitatively by 8M guanidinium hydrochloride and dialysis. In order to further purify the factors, any number of purification methods may be used, including but not limited to conventional ion exchange chromatography, affinity chromatography, different sugar chromatography, hydrophobic interaction chromatography, reverse phase chromatography or gel filtration.

When used herein, fusion polypeptide includes functionally equivalent molecules in which amino acid residues are substituted for residues within the sequence resulting in a silent or conservative change. For example, one or more amino acid residues within the sequence can be substituted by another amino acid of a similar polarity which acts as a functional equivalent, resulting in a silent or conservative alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Also included within the scope of the invention are proteins or fragments or derivatives thereof which exhibit the same or similar biological activity and derivatives which are differentially modified during or after translation, e.g., by glycosylation, proteolytic cleavage, linkage to an antibody molecule or other cellular ligand, etc.

Cells that express the fusion polypeptides of the invention are genetically engineered to produce them by, for example, transfection, transduction, electropration, or microinjection techniques.

In addition, the present invention contemplates use of the fusion polypeptides described herein in tagged form.

Any of the methods known to one skilled in the art for the insertion of DNA fragments into a vector may be used to construct expression vectors encoding the fusion polypeptides of the invention using appropriate transcriptional/translational control signals and protein coding sequences. These methods may include *in vitro* recombinant DNA and synthetic techniques and *in vivo* recombinations (genetic recombination). Expression of nucleic acid sequence encoding the fusion polypeptides of the invention may be regulated by a second nucleic acid sequence so that the fusion polypeptide is expressed in a host transformed with the recombinant DNA molecule. For example, expression of the fusion polypeptides described herein may be controlled by any promoter/enhancer element known in the art. Promoters which may be used to control expression of the fusion polypeptide include, but are not limited to the long terminal repeat as described in Squinto et al., (1991, Cell 65:1-20); the SV40 early promoter region (Bemoist and Chambon, 1981, Nature 290:304-310), the CMV promoter, the M-MuLV 5' terminal repeat the promoter contained in the 3'long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:144-1445), the regulatory sequences of the metallothionein gene (Brinster et al., 1982, Nature 296:39-42); prokaryotic expression vectors such as the β-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad: Sci. U.S.A. 80:21-25), see also "Useful proteins from recombinant bacteria" in Scientific American, 1980, 242:74-94; promoter elements from yeast or other fungi such as the Gal 4 promoter, the ADH (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58); alpha 1-antitrypsin gene control region which is active in the liver (Kelsey et al., 1987, Genes and Devel. 1:161-171), beta-globin gene control region which is active in myeloid cells (Mogram et al., 1985, Nature 315:338-340; Kollias et al., 1986, Cell 46:89-94); myelin basic protein gene control region which is active in oligodendrocyte cells in the brain (Readhead et al., 1987, Cell 48:703-712); myosin light chain-2 gene control region which is active in skeletal muscle (Shani, 1985, Nature 314:283-286), and gonadotropic releasing hormone gene control region which is active in the hypothalamus (Mason et al., 1986, Science 234:1372-1378).

Thus, according to the invention, expression vectors capable of being replicated in a bacterial or eukaryotic host comprising nucleic acids encoding a fusion polypeptide as described herein, and in particular modified angiopoietin, are used to transfect the host and thereby direct expression of such nucleic acid to produce fusion polypeptides which may then be recovered in biologically active form. As used herein, a biologically active form includes a form capable of binding to the relevant receptor and causing a differentiated function and/or influencing the phenotype of the cell expressing the receptor. Such biologically active forms would, for example, induce phosphorylation of the tyrosine kinase domain of Tie2 receptor, or stimulation of synthesis of cellular DNA.

Expression vectors containing the nucleic acid inserts can be identified by without limitation, at least three general approaches: (a) DNA-DNA hybridization, (b) presence or absence of "marker" gene functions, and (c) expression of inserted sequences. In the first approach, the presence of foreign nucleic acids inserted in an expression vector can be detected by DNA-DNA hybridization using probes comprising sequences that are homologous to an inserted nucleic acid sequences. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of foreign nucleic acid sequences in the vector. For example, if an *efl* nucleic acid sequence is inserted within the marker gene sequence of the vector, recombinants containing the insert can be identified by the absence of the marker gene function. In the third approach, recombinant expression vectors can be identified by assaying the foreign nucleic acid product expressed by the recombinant constructs. Such assays can be based, for example, on the physical or functional properties of the nucleic acid product of interest, for example, by binding of a ligand to a receptor or portion thereof which may be tagged with, for example, a detectable antibody or portion thereof or binding to antibodies produced against the protein of interest or a portion thereof.

The fusion polypeptide, in particular modified angiopoietin of the present invention, may be expressed in the host cells transiently, constitutively or permanently.

The invention contemplates the development of a fusion polypeptide as a therapeutic agent for the treatment of patients suffering from disorders involving cells, tissues or organs which express the Tie2 receptor. Such molecules may be used in a method of treatment of the human or animal body, or in a method of diagnosis.

Because Tie2 receptor has been identified in association with endothelial cells and, blocking of agonists of the receptor such as Ang-1 has been shown to prevent vascularization, applicants expect that Tie2 agonist fusion polypeptides may be useful for the induction of vascularization in diseases or disorders where such vascularization is indicated. Such diseases or disorders would include wound healing, ischemia and diabetes. The ligands may be tested in animal models and used therapeutically as described for other agents, such as vascular endothelial growth factor (VEGF), which is another endothelial cell-specific angiogenic factor.

U.S. Patent No. 5,332,671, as well as other studies, describe *in vitro* and *in vivo* studies that may be used to demonstrate the effect of an angiogenic factor in enhancing blood flow to ischemic myocardium, enhancing wound healing, and in other therapeutic settings wherein neoangiogenesis is desired. See also European Patent Application 0 550 296 A2; Banai, et al., Circulation 89:2183-2189 (1994); Unger, et al. Am.J.Physiol. 266:H1588-H1595 (1994); and Lazarous, et al. Circulation 91:145-153 (1995). The agonist fusion polypeptides may be used alone or in combination with one or more additional pharmaceutically active compounds such as, for example, VEGF or basic fibroblast growth factor (bFGF).

Conversely, antagonists of the Tie2 receptor, such as Tie2 receptorbodies or Ang-2 as described in Example 9 in WO 96/31598, have been shown to prevent or attenuate vascularization in certain situations and in certain amounts. Similarly, Tie2 antagonist fusion polypeptides would also be useful for those purposes. These antagonists may be used alone or in combination with other compositions, such as anti-VEGF antibodies, that have been shown to be useful in treating conditions in which the therapeutic intent is to block angiogenesis.

In other embodiments, the Tie2 agonist fusion polypeptides described herein may be used as hematopoietic factors. A variety of hematopoietic factors and their receptors are involved in the proliferation and/or differentiation and/or migration of the various cells types contained within blood. Because Tie2 receptors are expressed in early hematopoietic cells, the Tie2 ligands are expected to play a comparable role in the proliferation or differentiation or migration of these cells. Thus, for example, Tie2 agonist fusion polypeptide compositions may be prepared, assayed, examined in *in vitro* and *in vivo* biological systems and used therapeutically as described in any of the following: U.S. Patent No. 4,810,643; Lee, et al., Proc. Natl. Acad Sci. USA 82:4360-4364 (1985); Wong, et al. Science, 228:810-814 (1985); Yokota, et al. Proc. Natl. Acad. Sci (USA) 81:1070 (1984); WO 9105795; and WO 95/19985.

Accordingly, the fusion polypeptides may be used to diagnose or treat conditions in which normal hematopoiesis is suppressed, including, but not limited to anemia, thrombocytopenia, leukopenia and granulocytopenia. In a preferred embodiment, the fusion polypeptides may be used to stimulate differentiation of blood cell precursors in situations where a patient has a disease, such as acquired immune deficiency syndrome (AIDS), which is associated with reduction in normal blood cell levels, or in clinical settings in which enhancement of hematopoietic populations is desired, such as in conjunction, with bone marrow transplant, or in the treatment of aplasia or myelosuppression caused by radiation, chemical treatment or chemotherapy.

The fusion polypeptides of the present invention may be used alone, or in combination with other pharmaceutically active agents such as, for example, cytokines, neurotrophins, interleukins, etc. In a preferred embodiment, the fusion polypeptides may be used in conjunction with any of a number of factors which are known to induce stem. cell or other hematopoietic precursor proliferation, or factors acting on later cells in the hematopoietic pathway, including, but not limited to, hemopoietic maturation factor, thrombopoietin, stem cell factor, erythropoietin, G-CSF, CM-CSF and so on.

In an alternative embodiment, Tie2 receptor antagonist fusion polypeptides are used to diagnose or treat patients in which the desired result is inhibition of a hematopoietic pathway, such as for the treatment of myeloproliferative or other proliferative disorders of blood forming organs such as thrombocythemias, polycythemias and leukemias. In such embodiments, treatment may comprise use of a therapeutically effective amount of the fusion polypeptides as described herein.

Effective doses useful for treating those or other diseases or disorders may be determined using methods known to one skilled in the art (see, for example, Fingl, et al., The Pharmacological Basis of Therapeutics, Goodman and Gilman, eds. Macmillan Publishing Co, New York, pp. 1-46 (1975). Pharmaceutical compositions for use according to the invention includes the fusion polypeptides described above in a pharmacologically acceptable liquid, solid or semi-solid carrier, linked to a carrier or targeting molecule (e.g., antibody, hormone, growth factor, etc.) and/or incorporated into liposomes, microcapsules, and controlled release preparation prior to administration *in vivo*. For example, the pharmaceutical composition may comprise a fusion polypeptide in an aqueous solution, such as sterile water, saline, phosphate buffer or dextrose solution. Alternatively, the active agents may be comprised in a solid (e.g. wax) or semi-solid (e.g. gelatinous) formulation that may be implanted into a patient in need of such treatment. The administration route may be any mode of administration known in the art, including but not limited to intravenously, intrathecally, subcutaneously, intrauterinely, by injection into involved tissue, intraarterially, intranasally, orally, or via an implanted device.

Administration may result in the distribution of the active agent of the invention throughout the body or in a localized area. For example, in some conditions which involve distant regions of the nervous system, intravenous or intrathecal administration of agent may be desirable. In some situations, an implant containing active agent may be placed in or near the lesioned area. Suitable implants include, but are not limited to, gelfoam, wax, spray, or microparticle-based implants.

The present invention also contemplates pharmaceutical compositions comprising the fusion polypeptides described herein, in a pharmacologically acceptable vehicle. The compositions may be administered systemically or locally. Any appropriate mode of administration known in the art may be used, including, but not limited to, intravenous, intrathecal, intraarterial, intranasal, oral, subcutaneous, intraperitoneal, or by local injection or surgical implant. Sustained release formulations are also provided for.

**Gene Therapy**

In a specific embodiment, nucleic acids comprising sequences encoding the chimeric Ang1 polypeptide are administered to prevent vascular leakage, and for therapeutic vasoulogenesis, by way of gene therapy. Gene therapy refers to therapy performed by the administration to a subject of an expressed or expressible nucleic acid. In this embodiment, the nucleic acids produce their encoded protein that mediates a therapeutic effect.

Any of the methods for gene therapy available in the art can be used. Exemplary methods are described below.

For general reviews of the methods of gene therapy, see Goldspiel et al., Clinical Pharmacy 12:488-505 (1993); Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62: 191-217 (1993); May, TIBTECH 11(5):155-215 (1993). Methods commonly known in the art of recombinant DNA technology which can be used are described in Ausubel et aL (eds.), Current Protocols in Molecular Biology, John Wiley & Sons, NY (1993); and Kriegler, Gene Transfer and Expression, A Laboratory Manual, Stockton Press, NY (1990).

In a preferred aspect, nucleic acid sequences may encode a chimeric-Ang1 or Tie2 polypeptide, in which the nucleic acid sequences are part of expression vectors that express the polypeptides in a suitable host. In particular, such nucleic acid sequences have promoters operably linked to the polypeptide coding region, said promoter being inducible or constitutive, and, optionally, tissue-specific. In another particular embodiment, nucleic acid molecules are used in which the polypeptide coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA 86:8932-8935 (1989); Zijlstra et al., Nature 342:435-438 (1989).

Delivery of the nucleic acids into a patient may be either direct, in which case the patient is directly exposed to the nucleic acid or nucleic acid- carrying vectors, or indirect, in which case, cells are first transformed with the nucleic acids *in vitro*, then transplanted into the patient. These two approaches are known, respectively, as *in vivo* or *ex vivo* gene therapy.

In a specific embodiment, the nucleic acid sequences are directly administered *in vivo*, where it is expressed to produce the encoded product. This can be accomplished by any of numerous methods known in the art, e.g., by constructing them as part of an appropriate nucleic acid expression vector and administering it so that they become intracellular, e.g., by infection using defective or attenuated retrovirals or other viral vectors, or by direct injection of naked DNA, or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem. 262:4429-4432 (1987)) (which can be used to target cell types specifically expressing the receptors) and so on. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor. Alternatively, the nucleic acid can be introduced intracellularly and incorporated within host cell DNA for expression, by homologous recombination (Koller and Smithies, Proc. Natl. Acad. Sci. USA 86:8932-8935 (1989); Zijlstra et al., Nature 342:435-438 (1989)).

In a specific embodiment, viral vectors that contain nucleic acid sequences encoding the polypeptide are used. The nucleic acid sequences encoding the polypeptide to be used in gene therapy are cloned into one or more vectors, which facilitates delivery of the gene into a patient. Retroviral vectors, adenoviral vectors and adeno-associated viruses are examples of viral vectors that may be used. Retroviral vectors contain the components necessary for the correct packaging of the viral genome and integration into the host cell DNA.

Adenoviruses are especially attractive vehicles for delivering genes to respiratory epithelia because they naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. In addition, adeno-associated virus (AAV) has also been proposed for use in gene therapy.

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion and so on. Numerous techniques are known in the art for the introduction of foreign genes into cells and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as T-lymphocytes, B-lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, and so on.

In a preferred embodiment, the cell used for gene therapy is autologous to the patient.

In an embodiment in which recombinant cells are used in gene therapy, nucleic acid sequences encoding the polypeptide are introduced into the cells such that they are expressible by the cells or their progeny, and the recombinant cells are then administered *in vivo* for therapeutic effect. In a specific embodiment, stem or progenitor cells are used. Any stem and/or progenitor cells which can be isolated and maintained *in vitro* can potentially be used.

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

**Therapeutic Composition**

The formulation of therapeutic compounds is generally known in the art and reference can conveniently be made to Remington's Pharmaceutical Sciences, 17th ed., Mack Publishing Co., Easton, Pa., USA. For example, from about 0.05 µg to about 20 mg per kilogram of body weight per day may be administered. Dosage regime may be adjusted to provide the optimum therapeutic response. For example, several divided doses may be administered daily or the dose may be proportionally reduced as indicated by the exigencies of the therapeutic situation. The active compound may be administered in a convenient manner such as by the oral, intravenous (where water soluble), intramuscular, subcutaneous, intra nasal, intradermal or suppository routes or implanting (eg using slow release molecules by the intraperitoneal route or by using cells e.g. monocytes or dendrite cells sensitised *in vitro* and adoptively transferred to the recipient). Depending on the route of administration, the peptide may be required to be coated in a material to protect it from the action of enzymes, acids and other natural conditions which may inactivate said ingredients.

For example, the low lipophilicity of the peptides will allow them to be destroyed in the gastrointestinal tract by enzymes capable of cleaving peptide bonds and in the stomach by acid hydrolysis. In order to administer peptides by other than parenteral administration, they will be coated by, or administered with, a material to prevent its inactivation. For example, peptides may be administered in an adjuvant, co-admilistered with enzyme inhibitors or in liposomes. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEP) and trasylol. Liposomes include water-in-oil-in-water CGF emulsions as well as conventional liposomes.

The active compounds may also be administered parenterally or intraperitoneally. Dispersions can also be prepared in glycerol liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersion. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The prevention of the action of microorganisms can be brought about by various antibacterial and antifungal agents, for example, chlorobutanol, phenol, sorbic acid, theomersal and the like. In many cases, it will be preferable to include isotonic agents, for example, sugars or sodium chloride. Prolonged absorption of the injectable compositions can be brought about by the use in the composition of agents delaying absorption, for example, aluminium monostearate and gelatin.

Sterile injectable solutions are prepared by incorporating the active compounds in the required amount in the appropriate solvent with various other ingredients enumerated above, as required, followed by filtered sterilization. Generally, dispersions are prepared by incorporating the various sterile active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a previously sterile-filtered solution thereof.

When the peptides are suitably protected as described above, the active compound may be orally administered, for example, with an insert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions is such that a suitable dosage will be obtained. Preferred compositions or preparations according to the present invention are prepared so that an oral dosage unit form contains between about 0.1 µg and 2000 mg of active compound.

The tablets, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

**Delivery Systems**

Various delivery systems are known and can be used to administer a compound e.g., encapsulation in liposomes, microparticles, microcapsules, recombinant cells capable of expressing the compound, recoptor-mediated endocytosis, construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may bo desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions locally to the area in need of treatment; this may be achieved by, for examples, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody or a peptide of the invention, care must be taken to use materials to which the protein does not absorb. In another embodiment, the compound or composition can be delivered in a vesicle, in particular a liposome. In yet another embodiment, the compound or composition can be delivered in a controlled release system. In one embodiment, a pump may be used. In another embodiment, polymeric materials can be used. In yet another embodiment, a controlled release system can be placed in proximity of the therapeutic target, thus requiring only a fraction of the systemic dose.

Labels

Suitable enzyme labels include, for example, those from the oxidase group, which catalyze the production of hydrogen peroxide by reacting with substrate. Glucose oxidase is particularly preferred as it has good stability and its substrate (glucose) is readily available. Activity of an oxidase label may be assayed by measuring the concentration of hydrogen peroxide formed by the enzyme-labeled antibody/substrate reaction. Besides enzymes, other suitable labels include radioisotopes, such as iodine (¹²⁵I, ¹²¹I), carbon (¹⁴C), sulphur (³⁵S), tritium (³H), indium (¹¹²In), and technetium (^{99m}Tc), and fluorescent labels, such as fluorescein and rhodamine, and biotin.

Further suitable labels for the chimeric-Ang1, Tie2 or chimeric Ang1/Tie2 complex-specific antibodies of the present invention are provided below. Examples of suitable enzyme labels include malate dehydrogenase, δ-5-steroid isomerase, yeast-alcohol dehydrogenase, α-glycerol phosphate dehydrogenase, triose phosphate isomerase, peroxidase, alkaline phosphatase, asparaginase, glucose oxidase, β-galactosidase, ribonuclease, urease, catalyse, glucose-6-phosphate dehydrogenase, glucoamylase, and acetylcholine esterase.

Examples of suitable radioisotopic labels include ³H, ¹¹¹In, ¹²⁵I, ¹³¹I, ³²P, ³⁵S, ¹⁴C, ⁵¹Cr, ⁵⁷To, ⁵⁸Co, ⁵⁹Fe, ⁷⁵Se, ¹⁵²Eu, ⁹⁰Y, ⁶⁷Cu, ²¹⁷Ci, ²¹¹At, ²¹²Pb, ⁴⁷Sc, ¹⁰⁹Pd, etc. ¹¹¹In is preferred isotope where *in vivo* imaging is used since its avoids the problem of dehalogenation of the ¹²⁵I or ¹³¹I-labeled polypeptide by the liver. In addition, this radionucleotide has a more favorable gamma emission energy for imaging. For example, ¹¹¹In coupled to monoclonal antibodies with 1-(P-isothiocyanatobenzyl)-DPTA has shown little uptake in non-tumors tissues, particularly the liver, and therefore enhances specificity of tumor localization.

Examples of suitable non-radioactive isotopic labels include ¹⁵⁷Gd, ⁵⁵Mn, ¹⁶²Dy, 52^{Tr}, and ⁵⁶Fe.

Examples of suitable fluorescent labels include an ¹⁵²Eu label, a fluorescein label, an isothiocyanate label, a rhodamine label, a phycoerythrin label, a phycocyanin label, an allophycocyanin label, an o-phthaldehyde label, and a fluorescamine label.

Examples of suitable toxin labels include, Pseudomonas toxin, diphtheria toxin, ricin, and cholera toxin.

Examples of chemiluminescent labels include a luminal label, an isoluminal label, an aromatic acridinium ester label, an imidazole label, an acridinium salt label, an oxalate ester label, a luciferin label, a luciferase label, and an aequorin label.

Examples of nuclear magnetic resonance contrasting agents include heavy metal nuclei such as Gd, Mn, and iron. Deuterium may also be used. Other contrasting agents also exist for EPIT, PET or other imaging mechanisms, which are known to persons of skill in the art.

Typical techniques for binding the above-described labels to polypeptides are provided by Kennedy et aL (1976) Clin. Chim. Acta 70:1-31, and Schurs et al. (1977) Clin. Chim. Acta 81:1-40. Coupling techniques include the glutaraldehyde method, the periodate method, the dimaleimide method, the m-maleimidobonzyl-N-hydroxy-succinimide ester method.

The polypeptides of the present invention, including fragments thereof, may be used to detect chimeric-Ang1, Tie2 or chimeric Ang1/Tie2 complex using biochip and biosensor technology. Biochip and biosensors may comprise the polypeptides of the present invention to detect antibodies, which specifically recognize chimeric Ang1/Tie2 complex. Bio chip and biosensors may also comprise antibodies which specifically recognize the polypeptides of the present invention to detect "chimeric Ang1/Tie2 complex.
The following examples are offered by way of illustration of the present invention, and not by way of limitation.

### EXAMPLES

**EXAMPLE 1 - MULTIMERIZATION OF ANG1 FOR TIE2 PHOSPHORYLATION**

To determine the role of the amino-terminal region in relation to Tie2 receptor, truncated Ang1 that contains only fibrinogen-like domain (amino acids 284-498) (Angl/FD) was generated. Recombinant Ang1/FD was a ∼34 kDa secreted monomer, while recombinantly produced native Ang1 was a multimer (data not shown). Interestingly, the monomeric Ang1/FD did not bind to soluble Tie2, nor did it phosphorylate Tie2. In contrast, native Ang1 bound to Tie2 and also phosphorylated it. Thus, it appeared that both the amino terminus and multimerization of Ang1 could be necessary for Tie2 binding and activation.

To determined the role of Cys⁴¹, Cys⁵⁴ and Cys²⁶⁵ in Ang1 multimerization, the amino acid 17-80 region was deleted (Angl-D1) and Cys²⁶⁵ was substituted with Ser²⁶⁵ (Ang1S265). SDS-PAGE gel analysis revealed that recombinant Ang1-D1 was present as a trimer and Ang1S265 took on several types of multimeric formes including dimer and monomer. These data suggest that Cys⁴¹, Cys⁵⁴ and Cys²⁶⁵ of Ang1 participate in inter-and extra-molecular disulfide bond formation to form Ang1 multimers.

To determine the role of coiled-coil domains for Ang1 multimerization, we gradually deleted amino-terminal portion of Ang1. SDS-PAGE gel analysis revealed that deletion of amino acids 17-119 (Angl-D2), amino acids 17-153 (Angl-D3), and amino acids 17-212 (Angl-D4) resulted in the formation of trimer, and dimer. *In vitro* binding assay with soluble Tie2-Fc showed that Angl-D2, Angl-D3, and Angl-D4 bound to Tie2. Interestingly, trimeric Ang1-induced Tie2 phosphorylation was greater than dimeric Ang1-induced Tie2 phosphorylation. The data suggest that higher-order multimers induce greater level of Tie2 phosphorylation. In addition, the second coiled-coil domain (amino acids 153-261) and linker domain appear to be significant factors for creating higher-order multimerization. Moreover, multimerization of Ang1 is essential for Tie2 binding.

**EXAMPLE 2 - GENERATION OF GENE CONSTRUCTS FOR NATIVE ANG1**

The full cDNA of human Ang1 was amplified from human adult heart cDNA library (Clontech) by PCR for 30 cycles at an annealing temperature of 52°C using sense (5'-GTGCGGATTCACAATGACAGTTTTC-3' (SEQ ID NO:9), including BamHI restriction enzyme site; original 5'-GTGCGGCAGTACAATGACAGTTTTC-3' (SEQ ID NO:10)) and antisense primers (5'-GCTTTCAGATATCTAAAGGTCGAAT-3' (SEQ ID NO:11), including EcoRV restriction enzyme site; original 5'-GCTTTCAAAAATCTAAAGGTCGAAT-3' (SEQ ID NO:12)). The amplified DNA was cloned into the pCR-Blunt vector (Invitrogen) and sequence determined.

Thus obtained human Ang1 cDNA was re-subcloned into a CMV promoter-driven mammalian cell expression vector, pcDNA3.1/Myc-His (Invitrogen), which has a DNA fragment (63 bp) encoding c-myc and a 6X His tag at the 3'-terminus of the coding region as an open reading frame (CMV-Ang1-M-H).

To generate the recombinant Ang1 that has an N-terminal FLAG tag, PCR was performed on CMV-Ang1-M-H for 25 cycles at an annealing temperature of 60°C using sense primer (5'-CAGAAAAGCTTGGGAGAAGATAT-3' (SEQ ID NO:13)) and antisense primer (5'-TAGAAGGCACAGTCGAGGCTGA-3' (SEQ ID NO:14)). The PCR products were subcloned into cloning vector pCR2.1 (Invitrogen) and sequenced. The insert was cut with HindIII and PmeI, then subcloned into HindIII and EcoRV sites in pFLAG-CMV1 (Sigma). This vector was named 'native Angl'. EXAMPLE 3 - **GENERATION OF GENE CONSTRUCTS FOR CHIMERIC COILED-COIL CONTAINING ANG1**

In order to generate multimeric but smaller molecular weight Ang1, the amino-terminal portion of Ang1 (261 amino-acid) was replaced with the short coiled-coil domain of yeast transcriptional activator GCN4, cartilage matrix protein (CMP; matrilin or MAT), and cartilage oligomeric matrix protein (COMP). The coiled-coil domain of GCN4 is 31-amino acids and forms a parallel dimer. The coiled-coil domain of CMP is 43-amino acids and formes a parallel trimer. The coiled-coil domain of COMP is 45-amino acids and forms a parallel pentamer. Dr. Richard A. Kammerer (Department of Structural Biology, University of Basel) provided cDNA encoding coiled-coil domain of yeast GCN4, chicken MAT, and rat COMP.

PCR primers for coiled-coil domain of GCN4, CMP (MAT) and COMP including BglII and BamH1 restriction enzyme sites were designed.

GCN4 BglII, Sense primer: 5'-cagatcttaatgaaacagctggaagacaa-3' (SEQ ID NO:15).

GCN4 BamHI, Antisense primer: 5'-ttggatccttcaccaaccagttttttcagac-3' (SEQ ID NO:16).

CMP BglII, Sense primer: 5'- ccagatcttagaagaagatccgtgcgaatg-3' (SEQ ID NO:17).

CMP BamHI, Antisense primer: 5'- aaggatccgatgattttgttttccagcgc-3' (SEQ ID NO:18).

COMP BglII, Sense primer: 5'- ccagatcttagacctagccccacagatgct-3' (SEQ ID NO:19).

COMP BamHI, Antisense primer: 5'- ttggatcctccgcaagcgtcacattccatc-3' (SEQ ID NO:20).

PCR was performed for 30 cycles at an annealing temperature of 52°C. The PCR products were subcloned into cloning vector pZErO-2 (Invitrogen) and sequenced. It was named 'pZErO-2CCD'.

In order to generate the secretion signal sequence of hemagglutinin and FLAG including HindIII, BamHI and XhoI restriction enzyme sites, the following sense and antisense oligonucleotides were synthesized.

SHG-FLAG HindIII-BamH1-XhoI (HBX) Sense primer: 5'AAGCTTAAGCTTGCCACCATGAAGACGATCATCGCCCTGAGCTACATCTTCT GCCTGGTATTCGCCGACTACAAGGACGATGATGACAAGGGGATCCACTAGTCT CGAG-3' (SEQ ID NO:21).

SHG-FLAG XhoI-BamH1-HindIII (XBH) Antisense primer: 5'CTCGAGACTAGTGGATCCCCTTGTCATCATCGTCGTTGTAGTCGGCGAATAC CAGGCAGAAGATGTAGCTCAGGGCGATGATCGTCTTCATGGTGGCAAGCTTAA GCTT-3' (SEQ ID NO:22).

Sense and antisense nucleotides were annealed. The annealed reactants were ligated into the HindIII and XhoI sites of the mammalian cell expression vector, pCDNA3.1 (Invitrogen). This vector was named 'pCDNA-Signal-FLAG', which was incubated with BglII and BamHI. The released PCR fragment was inserted into BamH1 digested pCDNA-Signal-FLAG. This vector was named 'pCDNA-Signal-FLAG-CC'.

PCR primers for the linker and fibrinogen domain of Ang1 (from Leu261 to termination) including BamH1 and Xho1 restriction enzyme sites were designed.

A1LF BamH1, Sense primer: 5'- ttggatcccttgtcaatctttgcactaaag -3' (SEQ ID NO:23).

A1LF Xho1, Antisense primer: 5'-ttctcgagtcaaaaatctaaaggtcgaatcatc-3' (SEQ ID NO:24).

PCR was performed for 30 cycles at an annealing temperature of 52°C. The PCR products were subcloned into cloning vector pZErO-2 (In Vitrogen) and sequenced. It was named 'pZErO-2A1LF', which was incubated with BamHI and Xho1. The released PCR fragment was inserted into BamH1 and XhoI digested pCDNA-Signal-FLAG-CC. This vector was named 'pCDNA-Signal-FLAG-CC-A1LF'. Figure 6 shows a schematic diagram for generating gene constructs for the multimeric chimeric-Ang1.

As described *supra*, four gene constructs were made and confirmed by sequence analysis. See Figure 7.

(1) pCDNA-Signal-FLAG-human Ang1 (native Ang1).

(2) pCDNA-Signal-FLAG-coiled-coil domain of GCN4-fibrinogen domain of Ang1 (GCN4/CC-Ang1/FD; or GCN4-Angl)

(3) pCDNA-Signal-FLAG-coiled-coil domain of MAT-fibrinogen domain of Ang1 (CmP/CC-Ang1/FD; or MAT-Ang1).

(4) pCDNA-Signal-FLAG-coiled-coil domain of COMP-fibrinogen domain of Ang1 (COMP/CC-Angl/FD; or COMP-Ang1).

All of the above nucleic acid molecules were constructed by standard recombinant DNA techniques (See e.g., Molecular Cloning, A Laboratory Manual, Sambrook, et al., Cold Spring Harbor Laboratory, Cold Spring Harbor, NY; Current Protocols in Molecular Biology (Eds. Ausubel, et al., Greene Publ. Assoc., Wiley-Interscience, NY), sequence-verified by standard techniques using an ABI PRISM 3100 Genetic Analyzer (Applied Biosystems , Foster City, CA) and Taq Dideoxy Terminator Cycle Sequencing Kit using BigDye Terminator Cycle Sequencing version 2.0 (Applied Biosystems, Inc., Foster City, CA), ABI 373A DNA sequencer and subcloned into the mammalian expression vector pcDNA3.1 (Invitrogen, Inc.).

The bridging sequences described *infra* were introduced to provide convenient restriction sites and to give flexibility to the junctions between the domains, but there is no indication that there is a critical nature to these bridging sequences, though varying the length of the linker in some of these constructs led to some variation in the amount of protein produced.

**EXAMPLE 4: CONSTRUCTION OF NATIVE ANG1**

Native Ang1 consists of a preprotrypsin leader sequence (Met-Ser-Ala-Leu-Leu-Ile-Leu-Ala-Leu-Val-Gly-Ala-Ala-Ala (SEQ ID NO:25)) at its amino terminus to allow for secretion (bases 1-42), a FLAG tag sequence (Asp-Tyr-Lys-Asp-Asp-Asp-Asp-Lys (SEQ ID NO:26), bases 43-66), a bridging amino acid Leu (bases 67-69), the coding sequence of Ang1 (bases 70-1470), another bridging sequence consisting of the amino acids Asp-Ile-Gln-His-Ser-Gly-Gly-Arg-Ser-Ser-Leu-Glu-Gly-Pro-Arg-Phe (SEQ ID NO:27) (bases 1471-1518), and the Myc epitope (Glu-Gln-Lys-Leu-Ile-Ser-Glu-Glu-Asp-Leu (SEQ ID NO:28), bases 1519-1548), and a short bridging sequence consisting of amino acids Asn-Met-His-Thr-Gly (SEQ ID NO:29) (bases 1549-1563) followed by His-Tag (His-His-His-His-His-His (SEQ ID NO:30), bases 1564-1581).

**EXAMPLE 5: CONSTRUCTION OF THE GCN4/CC-ANG1/FD**

GCN4/CC-Ang1/FD consists of a hemagglutinin signal sequence at its amino terminus to allow for secretion (bases 1-48 of SEQ ID NO:3 (Figure 3A), a FLAG tag sequence (bases 49-72 of SEQ ID NO:3 (Figure 3A)), a short bridging sequence consisting of the amino acids Gly-Ile-Leu of SEQ ID NO:3 (Figure 3A)), the coding sequence of GCN4 coiled-coil domain (bases 82-174 of SEQ ID NO:3 (Figure 3A)), another bridging sequence of the amino acids Gly-Ser (bases 175-180 of SEQ ID NO:3 (Figure 3A)), and the coding sequence for the linker region of Ang1 (bases 181-249 of SEQ ID NO:3 (Figure 3A)) followed by fibrinogen domain of Ang1 (FD) (bases 250-897 of SEQ ID NO:3 (Figures 3A-3C)).

**EXAMPLE 6: CONSTRUCTION OF THE CMP/CC-ANG1/FD**

CMP/CC-Ang1/FD consists of a hemagglutinin signal sequence at its amino terminus to allow for secretion (bases 1-48 of SEQ ID NO:5 (Figure 4A)), a FLAG tag sequence (bases 49-72 of SEQ ID NO:5 (Figure 4A)), a short bridging sequence consisting of the amino acids Gly-Ile-Leu (bases 73-81 of SEQ ID NO:5 (Figure 4A)), the coding sequence of MAT coiled-coil domain (bases 82-210 of SEQ ID NO:5 (Figure 4A)), another bridging sequence consisting of the amino acids Gly-Ser (bases 211-216 of SEQ ID NO:5 (Figure 4A)), and the coding sequence for the linker region of Ang1 (bases 217-285 of SEQ ID NO:5 (Figure 4A)) followed by fibrinogen domain of Ang1 (FD) (bases 286-933 of SEQ ID NO:5 (Figures 4A-4C)).

**EXAMPLE 7: CONSTRUCTION OF THE COMP/CC-ANG1/FD**

COMP/CC-Angl/FD consists of a hemagglutinin signal sequence at its amino terminus to allow for secretion (bases 1-48 of SEQ ID NO:7 (Figure 5A)), a FLAG tag sequence (bases 49-72 of SEQ ID NO:7 (Figure 5A)), a short bridging sequence consisting of the amino acids Gly-Ile-Leu (bases 73-81 of SEQ ID NO:7 (Figure 5A)), the coding sequence of COMP coiled-coil domain (bases 82-221 of SEQ ID NO:7 (Figure 5A)), another bridging sequence consisting of amino acids Gly-Ser (bases 222-227 of SEQ ID NO:7 (Figure 5A)), and the coding sequence for the linker region of Ang1 (bases 228-296 of SEQ ID NO:7 (Figure 5A)) followed by fibrinogen domain of Ang1 (FD) (bases 250-949 of SEQ ID NO:7 (Figures 5A-5C)).

**EXAMPLE 8: CHARACTERIZATION OF CHIMERIC-ANG1 PROTEIN - MOLECULAR WEIGHT ANALYSIS**

The predicted molecular weights for native Ang1, Angl/FD, CC-Ang1, GCN4-Ang1, MAT-Ang1 and COMP-Ang1 under reduced state were determined using the Swiss-PROT Program. Predicted weights of FLAG-tagged native Ang1, GCN4-Angl, MAT-Ang1 and COMP-Ang1 are 58810.82 Da, 32044.28 Da, 33330.88 Da and 33522.96 Da, respectively. There are five N-linked glycosylation sites in native Ang1, while there is one predicted N-linked glycosylation site in each of GCN4-Angl, MAT-Ang1 and COMP-Ang1. These predicted N-linked glycosylation sites could potentially increase the molecular weight by approximately 2500 Da/site. Thus, the predicted molecular weights for native Ang1, GCN4-Ang1, MAT-Ang1 and COMP-Ang1 under reducing conditions are approximately, 71.3 kDa, 34.5 kDa, 35.8 kDa, and 36.0 kDa, respectively. Subsequent SDS PAGE analyses (Figure 8) of COS cell-derived protein described *infra* confirmed these approximate molecular weights. Molecular weights of native Ang1 (∼75 kDa), GCN4-Angl (∼40 kDa), MAT-Ang1 (∼43 kDa), and COMP-Ang1 (∼44 kDa) were determined under reducing conditions.

Variable sizes of native Ang1 and the expected size of GCN4-Angl (∼80 kDa) were shown under non-reducing conditions. However, MAT-Ang1 (∼130 kDa and ∼180 kDa) and COMP-Ang1 (∼180 kDa and ∼220 kDa) exhibited an unexpectedly large molecular weight under non-reducing conditions as compared with the expected molecular weights of recombinant MAT-Ang1 (∼135 kDa) and COMP-Ang1 (∼220 kDa) were shown under the non-reducing condition (Figure 8).

**EXAMPLE 9 - EXPRESSION LEVEL IN COS CELLS**

Recombinant proteins of native Ang1, GCN4-Ang1, MAT-Ang1, and COMP-Ang1 were assayed by transient expression in COS-7 cells (American Type Culture Collection, Manassas, VA) using Effectene (a liposome) transfection method according to manufacturer's instructions (Qiagen, Inc.). Briefly, COS-7 cells were grown on gelatin-coated 100 mm dishes with Dulbecco's modified Eagle's medium (DMEM) with 10% FBS at 37°C in 5% CO₂ atmosphere. About 40-50% confluent dishes of COS-7 cells were used for transfection. Effectene transfection reagents were mixed with DNA-Enhancer mixture. After 10 min incubation for transfection-complex formation, the transfection complexes were added onto the cells and the cells were incubated with DMEM with 5% FBS at 37°C in 5% CO₂ atmosphere. The supernatant was harvested from transfected cells after 48-60 hr.

**EXAMPLE 10 - PURIFICATION OF COS CELL LINE SUPERNATANT**

Because the recombinant proteins contain FLAG sequence, purification is relatively simple and straightforward using anti-FLAG M1 antibody-agarose affinity gel column chromatography (Sigma-Aldrich, Inc.). Briefly, anti-FLAG M1 antibody-agarose gel was washed with 0.1 M glycine/HCl (pH 3.5) and equilibrated with 1X TBS buffer (50 mM Tris, 150 mM NaCl, pH 7.4). The supernatant containing each recombinant protein was passed through a column filled with anti-FLAG M1 antibody-agarose gel. After triple passage of the supernatant, the column was washed with 1X TBS buffer containing 1 mM CaCl₂. The recombinant protein bound to the M1 gel was eluted with elution buffer containing 1X TBS and FLAG peptide (Sigma-Aldrich, Inc.). The relatively easy purification of the recombinant proteins provides a distinct advantage over purifying the parent protein, angiopoietin-1, which requires a highly extensive and labor-intensive purification scheme. After purification of COS-7 supernatants, recombinant proteins were quantitated using conventional Bradford method with DU 800 spectrophotometer (Beckman, Inc.) and confirmed with Coomassie blue staining of SDS-PAGE gel. These analyses showed that 600-700 µg of each recombinant protein/liter of COS-7 cell supernatant was obtained, which represents moderate level of expression.

The recombinant proteins were efficiently secreted from COS-7 cells with more than 98% of the total proteins present in the culture medium. All recombinant proteins bound with high efficiently (>95%) to their respective purification matrices. Approximately 40-50% of the recombinant native Ang1 was recovered from the purification matrix, while approximately 80% of the GCN4-, MAT-, and COMP-Ang1 variants were recovered. A solubility assay with acid precipitation indicated that GCN4-Ang1, MAT-Ang1, and COMP-Ang1 have high solubility, but native Ang1 has a low solubility (Table 1). Furthermore, GCN4-Angl, MAT-Ang1, and COMP-Ang1 are very stable and do not aggregate over time, whereas native Ang1 progressively lost its activity and aggregated over time (Table 1).

**Table 1 Biophysical characteristics of chimeric angiopoietin-1 variants**

| | Elution (%) | Solubility (%) | Stability (%) |
|---|---|---|---|
| Native Ang1 | 40-50 | 60-70 | 60-70 |
| GCN-Ang1 | >80 | >95 | >95 |
| CMP-Ang1 | >80 | >95 | >95 |
| COMP-Ang1 | >80 | >95 | >95 |

| | | | |
|---|---|---|---|
| Elution (%): from anti-FLAG Ab conjugated agarose bead using FLAG peptide Solubility (%): acid and alkaline precipitation test Stability (%): storage at -70°C for 3 months - multimerization and activities | | | |

SDS-PAGE analysis of the purified proteins under denaturing conditions revealed predominantly single bands of the expected molecular masses (Fig. 8A). Under non-reducing conditions, full-length Ang1 formed characteristic disulfide-linked multimers of various sizes, while GCN4-Angl formed disulfide-linked dimers, MAT-Ang1 formed disulfide-linked trimers and tetramers, and COMP-Ang1 formed disulfide-linked tetramers and pentamers (Fig. 8A). To confirm the oligomeric structure of native and chimeric Ang1 variants, we used rotary shadowing transmission electron microscopy (TEM) to directly image the molecules.

*Materials and Methods -* Glycerol spraying/low-angle rotary metal-shadowing and TEM - For glycerol spraying/low-angle rotary metal-shadowing, 20 µl of protein samples, 0.02-0.1 mg/ml in TBS, plus 30% glycerol, were sprayed onto freshly cleaved mica at room temperature and rotary shadowed in a BA 511 M freeze-etch apparatus (Balzers) with platinum/carbon at an elevation angle of 3-5° (36). Electron micrographs were taken in a Philips Morgagni TEM operated at 80 kV equipped with a Megaview III CCD camera.

Approximately 20-25% of the images obtained for native Ang1 showed the molecule in a trimeric state yielding mushroom-shaped structures, consisting of an irregular bulbous 'cap' structure (binding domain) at the end of a 'stalk' structure (4-stranded coiled-coil) with a small globular structure (clustering domain) at the other end of the 'stalk' (Fig. 8B). The majority of the images (75-80%) obtained for native Ang1 showed variations on the 'mushroom-like' building block, with the basic trimers coming together by clustering at a central point (Fig. 8B). These data indicate that recombinant native Ang1 has a basic trimeric structure with heterogeneity in the degree of multimerization. These results are essentially consistent with results that were obtained by Davis and colleagues (Davis, S. et al. 2003, Nat. Struct. Biol. 10:38-44), although they conclude that their basic unit of Ang1 is a dimer which is unlikely for an amino-terminal coiled-coil domain. Nevertheless, variations in the activity of native Ang1 preparations could be a result of its degree of multimerization. In comparison, the most frequent images obtained for GCN4-Angl, CC-Ang1 and COMP-Ang1 were dimers (>95%), trimer (>95%) and pentamers (∼65%), respectively. Interestingly, MAT-Ang1 seemed to form a tetramer (∼70%) rather than a trimer that is consistent with the gel data (Fig. 8B).

COS-7 cell supernatant yielded approximately 1 mg each of purified native Ang1, GCN4-Ang1, MAT-Ang1 and COMP-Ang1 that were used in the studies described *infra* to further characterize the protein.

**EXAMPLE 11 - RECEPTOR BINDING ANALYSIS OF COS CELL-DERIVED CHIMERIC-ANG1**

Binding analysis of GCN4-Angl, MAT-Ang1, and COMP-Ang1 to soluble Tie2-Fc receptor was performed using *in vitro* binding assay and Biacore assay. *In-vitro* standard binding assays revealed that GCN4-Ang1, MAT-Ang1, and COMP-Ang1 bind to soluble Tie2 receptor (Figure 9).

Twenty nanograms of each recombinant chimeric-Ang1 protein and 100 ng of soluble Tie1-Fc or soluble Tie2-Fc were incubated in 500 µl Tris-buffer solution (50 mM Tris, 100 mM NaCl, pH 7.4) containing 0.02% TritonX-100 at 4 °C for 2 hr. Then, 20 µl of protein-A agarose beads (Oncogene) was added and incubated for another 1 hr at 4 °C. The protein-A conjugated samples were washed twice with 1 ml of Tris-buffer containing 0.02% TritonX-100. The samples were eluted with sample buffer, and heat-denatured. The samples were further separated by 10% SDS-PAGE, and electro-blotted on to nitrocellulose membranes, and Western blotted with anti-FLAG M1 antibody to detect the bound recombinant chimeric-Ang1, and further washed and incubated with horseradish peroxidase-conjugated secondary antibody. Signal was visualized by chemiluminescent detection according to the manufacturer's protocol (Amersham Pharmacia Biotech) using chemilummescence scanner (LAS-1000, Fuji Film, Tokyo). See Figure 9. Approximately 75%, 20%, 95% or 100% of native Ang1, GCN4-Angl, MAT-Ang1, and COMP-Ang1, respectively, bound to sTie2-Fc. In contrast, none of them bound to sTie1-Fc.

Alternatively, to determine whether the COS-7 cell-derived recombinant proteins of native Ang1, GGN4-Ang1, MAT-Angland COMP-Ang1 could bind to Tie-2 receptor, standard Biacore analysis was performed by BIA2000 (BIAcore, Inc.). Briefly, 600 ng of Tie-2-Fc receptor protein (R&D Inc), which is a fusion protein composed of the ectodomain of Tie-2 receptor and Fc domain of human IgG1, was immobilized on a Biacore chip (Sensor Chip CM5). As a control, 600 ng of Fc protein, which had only the Fc domain of human IgG1, was also immobilized on the same chip. The binding affinity was obtained by subtracting the response value using Fc protein from the values obtained using the Tie-2-Fc protein.

The recombinant proteins including native Ang1, GCN4-Angl, MAT-Ang1 and COMP-Ang1 were passed over the chip to allow binding between Tie-2 receptor ectodomain and the recombinant proteins. The binding step was followed by a dissociation step that allowed the bound proteins to dissociate from the Tie-2 receptor ectodomain. GCN4-Ang1 was completely dissociated from the immobilized Tie-2 receptor ectodomain within 5 minutes, while MAT-Ang1, COMP-Ang1, and native Ang1 was not as easily dissociated, implying that there is a strong interaction between MAT-Ang1, COMP-Ang1, native Ang1 and Tie-2 receptor ectodomain. To dissociate these proteins from the receptor, various HCl solutions (pH 4.0 - 2.0) and high salt (1M NaCl) solution were passed over the chip. However, these solutions were not able to disrupt the interaction between Tie-2 receptor ectodomain and the recombinant proteins, indicating that there is a strong interaction between the Tie-2 receptor ectodomain and the recombinant proteins (MAT-Ang1, COMP-Ang1, and native Ang1) See Figures 10A-10D and 11.

In addition, association phase of native Ang1 was slower than GCN4-Angl/FD, 1MAT-Ang1/FD, COMP-Angl/FD, implying that modification of coiled-coil domain of the native Ang1 increased its association constant to Tie2 receptor, and that high affinity of the native Ang1 came from its slow dissociation.

**EXAMPLE 12 - TIE2 PHOSPHORYLATION ASSAY**

Human umbilical vein endothelial cells (HUVECs) were prepared from human umbilical cords by collagenase digestion and maintained as previously described (Kim et al., 2000 Circ. Res. 86: 24-29). The primary cultured cells used for the biochemical assays were between passages 2 and 3. Primary cultured HUVECs were incubated with control buffer, and 200 ng of native Ang1, and 100 ng of GCN4-Ang1, MAT-Ang1, or COMP-Ang1 for 10 min. The cells were harvested in extraction buffer, and 0.5 mg of protein was used for immunoprecipitation. Tie2 proteins in the sample were immunoprecipitated with anti-Tie2 antibody and collected. Immunoprecipitated samples were Western blotted with anti-phospho-tyrosine antibody (Figure 12A, upper band), and the membrane was re-blotted with anti-Tie2 antibody (Figure 12A, lower band) to verify equal loading of protein in each lane. The assay revealed that COMP-Ang1-induced Tie2 phosphorylation (∼8.8 fold) is much higher than phosphorylation induced by native Ang1 (∼3.6 fold) or MAT-Ang1 (∼4.0 fold). However, GCN4-Angl did not change Tie2 phosphorylation (Figure 12).

**EXAMPLE 13 - AKT (SER473) PHOSPHORYLATION ASSAY**

HUVECs were incubated with control buffer, and 200 ng of native Ang1, GCN4-Angl, MAT-Ang1 or COMP-Ang1 for 15 min. Alternatively, HUVECs were incubated with control buffer and different amounts of COMP-Ang1. HUVECs were also incubated with 200 ng of COMP-Ang1 for different times. After treatment, cell lysates were harvested. Each lane contained 50 µg of total protein from the cell lysates. Western blots were probed with anti-phospho-Akt (Ser473) antibody. The blot was reprobed with anti-Akt antibody (Figure 13A-13C, lower bands) to verify equal loading of protein in each lane. The assay revealed that COMP-Ang1-induced Akt (Ser 473) phosphorylation (9.7 fold) is greater than Akt (Ser 473) phosphorylation induced by native Ang1 (3.7 fold) or MAT-Ang1 (3.3 fold). However, GCN4-Angl does not change Akt (Ser 473) phosphorylation. COMP-Ang1-induced Akt (Ser 473) phosphorylation occurs in a dose-dependent manner and persists up to 60 min (Figures 13A-13C, upper bands).

**EXAMPLE 14 - APOPTOSIS ASSAY**

To induce apoptosis, HUVECs were plated onto gelatinized 24-well plates (7 x 10⁴ cells per well) in M-199 containing 20% FBS and incubated for 12 hr. The wells were extensively washed with PBS, and the medium was changed to serum-free M-199 containing control buffer, 200 ng/ml of native Ang1, GCN4-Angl, MAT-Ang1, or COMP-Ang1, and incubated for 30 hr. Floating apoptotic cells were collected with 2 washes in PBS. Adherent cells were collected by trypsinization. All cells were stained with Annexin V FLUOS staining kit (Roche Molecular Biochemicals, Mannheim, Germany) for 15 min at 20°C. Following staining of Annexin-V and propidium iodide (PI), the cells were analyzed on a flow cytometer and data were analyzed with CellQuest software (Becton Dickinson). The results were: Native Ang1 (about 47% increased cell survival), GCN4-Angl (about 12% increased cell survival), MAT-Ang1 (about 55% increased cell survival) and COMP-Ang1 (about 71% increased cell survival). Figures 14A-14E. See also Table 2.

**Table 2. Biological activities of native and chimeric Ang1 proteins in endothelial cells.**

| | Apoptosis | Migration | Tube length | Sprouting |
|---|---|---|---|---|
| **Vector** | 32.4 ± 5.6 | 56.4 ± 3.5 | 28.4 ± 4.2 | 35.5 ± 7.5 |
| **Native Ang1** | 17.2 ± 2.3 | 104.7 ± 4.6 | 42.4 ± 4.8 | 91.5 ± 10.5 |
| **GCN4-Ang1** | 28. 6 ± 4.3 | 68.0 ± 7.2 | 25.6 ± 3.3 | 42.5 ± 8.6 |
| **MAT-Ang1** | 14.7 ± 2.2 | 116.2 ± 4.2 | 43.6 ± 4.6 | 78.8 ± 8.8 |
| **COMP-Ang1** | 9.4 ± 1.4 | 145.5 ± 6.3 | 55.8 ± 6.9 | 121.5 ± 11.5 |

**EXAMPLE 15 *-IN VITRO* WOUNDING MIGRATION ASSAY**

Cell migration assay was carried out according to Sato and Rifkin (1989, J.Cell.Biol. 109:309-315) with slight modification. Primary cultured HUVECs were grown to confluence in 30 mm diameter dish in 1 ml of normal growth medium. Wounds were made in the monolayer by scratching the cell layer with a double-edged razor blade and the injury line marked. The scratch extended over an area 5-7 mm wide. After wounding, the cultures were washed immediately with serum-free medium to remove cell debris and any soluble factors that had been released. The wounded cells were further incubated in serum free medium with control buffer or 200 ng/ml of native Ang1, GCN4-Ang1, MAT-Ang1, or COMP-Ang1. HUVECs were allowed to migrate for 10 hr and were rinsed with PBS, followed by fixing with absolute methanol and staining with Giemsa. Migration was quantitated by counting the number of cells that moved beyond the reference line. All experiments were performed in triplicate. The results were: Native Ang1 (about 1.86 fold increase of cell migration), GCN4-Ang1 (about 1.20 fold increase of cell migration), MAT-Ang1 (about 2.06 fold increase of cell migration) and COMP-Ang1 (about 2.58 fold increase of cell migration). See Figures 14F-14J. See also Table 2.

**EXAMPLE 16 - TUBE FORMATION ASSAY**

Matrigel (Sigma-Aldrich Inc.) was thawed overnight at 4 °C and mixed to homogeneity using cooled pipette tips. Matrigel was added to the 24-well tissue culture plate (250 µl/well) at 4°C. The 24-well plate was brought to a 37°C cell culture incubator and incubated for 1 hr to allow the Matrigel to solidify. HUVECs were trypsinized, counted, resuspended in serum free M-199 medium, and added on Matrigel (lx 10⁵ cells/well) in the presence of control buffer, and 200 ng/ml of native Ang1, GCN4-Angl, MAT-Ang1 or COMP-Ang1. Cells were incubated for 12 hr to allow capillary-like structure to form. After 12 hr, the wells were washed with PBS, fixed for 30 min in 0.5% glutaraldehyde, and the length of capillary-like tubes were quantified using Image Pro-Express Software (CyberMedia). The results were: Native Ang1 (about 1.49 fold increase of tube formation), GCN4-Angl (about 0.90 fold increase of tube formation), MAT-Ang1 (about 1.53 fold increase of tube formation) and COMP-Ang1 (about 1.96 fold increase of tube formation). See Figures 14K-14O. See also Table 2.

**EXAMPLE 17 - SPROUTING ASSAY**

Cell sprouting assay in porcine pulmonary artery endothelial cells (PPAECs) was performed as previously described (Kim et al., 2000, Circ. Res. 86:952-959). Briefly, PPAECs were grown to confluence on microcarrier beads (diameter 175 µm; Sigma) and placed in a 2.5 mg/ml fibrinogen gel containing 2.0% heat-inactivated FBS and the indicated recombinant protein. Fibrin gels were incubated in DMEM with a daily addition of the same amount of recombinant protein. After 3 days, two independent investigators with no knowledge of which is the experimental or control counted the number of sprouts using an inverted microscope. The number of endothelial sprouts with length exceeding the diameter of the microcarrier beads (175 µm) per 50 microcarrier beads was counted. Inter-investigator variation was <5%. The mean number from the two investigators was used to estimate the number of sprout formation. The results were: Native Ang1 (about 2.58 fold increase of sprouting), GCN4-Angl (about 1.20 fold increase of sprouting), MAT-Ang1 (about 2.22 fold increase of sprouting) and COMP-Ang1 (about 3.42 fold increase of sprouting). See Figures 14P-14T. See also Table 2.

**EXAMPLE 18** - COMP-Ang1 alone induces strong angiogenesis *in vivo*.

To assess the effect of native Ang1 and COMP-Ang1 on *in vivo* angiogenesis, we performed a corneal micropocket assay with implantation of pellets containing the recombinant proteins.

*Materials and Methods -* Eight-week old male C57BL/6J mice (Jackson Labs, Bar Harbor, Maine) were used for all experiments. Preoperative anesthesia was by way of an intraperitoneal injection of pentobarbital (160 mg/kg). The eyes were anesthetized with 0.5% proparacaine (Alcaine, Alcon, Belgium). Using an operating microscope (SZ4045TRPT, Olympus, Japan), a central, intrastromal linear keratotomy approximately 0.6 mm length was performed with a surgical blade (Bard-Parker no. 15; Becton Dickenson, Franklin Lakes, New York), and a micropocket was dissected toward the temporal limbus using a modified von Graefe knife (Muthukkaruppan, V. & Auerbach, R. 1979, Science 205:1416-1418). The pocket was extended to within 1.0 mm of the temporal limbus (Kenyon, B.M. et al. 1996, Invest. Ophthalmol. Vis. Sci. 37:1625-1632). A sucrose aluminum sulfate pellet (0.4 x 0.4 x 0.2 mm; Sigma-Aldrich) was coated with hydron polymer (Sigma-Aldrich) containing one of the following: control buffer (0.05 mol/L Tris-HCl, pH 7.5, 150 mmol/L NaCl, 0.05% Chaps); native Ang1 (600 ng/pellet); or COMP-Ang1 (300 ng/pellet). The pellet was positioned 0.5 mm from the corneal limbus, and erythromycin ophthalmic ointment was applied to each operated eye. On postoperative day 6 after pellet implantation, the corneas of all mice were examined by a Coolpix995 digital Camera (Nikon, Japan) attached to a SZ4045TRPT stereomicroscope (Olympus, Japan) and photographed. Vessel length and the arc of corneal circumference occupied by angiogenesis (circumferential angiogenesis, in degrees) were measured. After these measurements were completed, mice received an i.v. injection of 500 µg of the endothelial cell-specific marker BS-1 lectin conjugated to FITC (Vector Laboratories, Burlingame, California). After 30 minutes, the animals were killed. The eyes were enucleated and fixed in 1% paraformaldehyde. After fixation, the corneas were placed on glass slides and examined by an AxioCAM CCD camera attached to an Axioplan2 Imaging fluorescence microscopy (Carl Zeiss, Germany).

On the sixth day after pellet implantation, corneal angiogenesis was evaluated using a stereomicroscope (Fig. 16A). Consistent with a previous report (Asahara, T. et al. 1998, Circ. Res. 83:233-240), the pellet containing control buffer or native Ang1 alone did not induce significant angiogenesis. However, the pellet containing COMP-Ang1 induced corneal angiogenesis extending from the limbus across the cornea (0.96 ± 0.22 mm in length) (Fig. 16A-16D). Thus, COMP-Ang1 shows a potent angiogenic effect *in vivo*. We also measured the luminal diameter of the corneal limbus arteries using *in situ* BS-1 lectin fluorescent microscopy in the same animals (Fig. 16B, left panels). Interestingly, COMP-Ang1 increased the luminal diameter of the basal limbus artery, while native Ang1 did not produce any change (Fig. 16D). Histological analysis of corneal sections showing the anterior portions of the limbal artery confirmed that angiogenesis was active in COMP-Ang1-treated corneas compared to control- and native Ang1-treated corneas (Fig. 16B, right panels). These data suggest that COMP-Ang1 alone can produce an angiogenic effect, with increased blood flow through more and larger arteries.

**EXAMPLE** 19 - Acute injection of COMP-Ang1 induces Tie2 phosphorylation in lung.

Examination of tissue expression of Tie2 in adult mouse revealed that the protein is most abundant in the lungs (Fig. 17A). Immunohistochemical analysis revealed that Tie2 is expressed in most endothelial cells in large vessels and microcapillaries, as evidenced by immunostaining to endothelial cell surface marker PECAM-1 (Fig. 17A-F). Therefore, we examined the effect of acute administration of native Ang1 and COMP-Ang1 on Tie2 activation in the lung of adult mice. Tie2 phosphorylation induced by COMP-Ang1 was not only greater, but also significantly more persistent than that of native Ang1 (Fig. 17B-17C). Consistent results were also obtained for the phosphorylation of Akt (data not shown). Thus, COMP-Ang1 is more potent than native Ang1 for activation of Tie2 *in vivo*. Moreover, these data imply that acute administration of COMP-Ang1 could activate Tie2 in most endothelial cells *in vivo*.

**EXAMPLE** 20 - COMP-Ang1 protects against radiation-induced apoptosis and death.

Damage to the gastrointestinal (GI) tract limits the use of radiation in cancer treatment. Microvascular endothelial apoptosis in the intestinal villi is the primary lesion caused by high doses of radiation (Paris, F. et al. 2001, Science 293:293-297). Therefore, we examined the effect of intravenous (i.v.) treatment with COMP-Ang1 on microvascular endothelial survival in a murine whole-body irradiation model.

*Materials and Methods* - Radiation was delivered with a 6 mev linear accelerator (NELAC-1006X, Tokyo, Japan) at a dose of 12-15 Gy. For protein injection, mice were injected iv with 100 µg COMP-Ang1 in 33-µg doses delivered 30 min before, 30 min after, and 90 min after irradiation. For control mice, 100 µg BSA was injected at the same time. Survival was monitored every 12 hr after irradiation. Actuarial survival was calculated by the product limit Kaplan-Meier method (Kaplan, E. & Meier, P. 1958, J. Am. Statist. Assoc. 53:457-816), and P values were evaluated by two-tailed Fisher's exact test.

*Immunohistochemistry and apoptosis assay -* Upon killing of mice, the tissues were frozen in OCT in methyl-butane (Sigma-Aldrich) on dry ice or were fixed in 10% neutral buffered formalin and embedded in paraffin. Frozen tissue blocks were sectioned at 10 µm and the sections were incubated with anti-Tie2 antibody (C-20, Santa Cruz Biotechnology, Santa Cruz, CA) or anti-PECAM-1 antibody (MEC13.3, BD PharMingen, San Diego, California) at 4°C overnight. Signals were visualized with the Cell and Tissue Staining Kit (R&D Systems). Sections were counterstained with methyl green or Meyer's hematoxylin. Paraffin tissue blocks in corneas were sectioned at 5 µm and stained with hematoxylin and eosin. Paraffin tissue blocks in irradiated tissues were sectioned 8 µm thick and were co-stained with PECAM-1 and TUNEL method (Intergen, Norcross, Georgia) to detect endothelial apoptotic cells. The microcapillary endothelial cells and apoptotic cells in the small intestinal villi and lung endothelium were viewed, counted, and photographed with an Axioskope2 plus microscope (Carl Zeiss, Germany) equipped with color CCD camera (ProgResC14, Jenoptik, Germany) and monitor. Two independent, blinded investigators counted apoptotic endothelial cells in approximately 1,000 villi from 5 different animals (approximately 200 villi per mouse) for each group. Inter-investigator variation was <5%.

*Statistics* - Data are expressed as mean ± standard deviation. Statistical significance was tested using one-way ANOVA followed by the Student-Nowman-Keuls test. Statistical significance was set at p<0.05.

Terminal deoxynucleotidyl transferase-mediated dUTP nick-end labeling (TUNEL) of intestinal specimens of FVB mice 4 hrs after irradiation (15 Gy) showed maximal and extensive endothelial apoptosis in villi, which is consistent with a previous report (Paris, F. et al. 2001, Science 293:293-297). IV injection of COMP-Ang1 into FVB mice immediately before and after irradiation with 15 Gy dramatically reduced apoptotic endothelial damage, but did not affect apoptosis of intestinal epithelial cells and crypt cells (Fig. 18A-18H). Although we found extensive apoptosis in thymus, spleen, salivary gland cells, rectal cryptic cells, lymphoid tissues, and polymorphonuclear cells in blood after irradiation exposure, the treatment with COMP-Ang1 had no significant effect on these non-endothelial cells. Thus, COMP-Ang1 provides strong protection against irradiation damage specifically to endothelial cells. Accordingly, the actual survival of 15 Gy-irradiated mice increased from a mean of 144 ± 7 hrs (median 144 hrs) in control mice to 190 ± 8 hrs (median 192 hrs) in mice treated with i.v. COMP-Ang1 (Fig. 18I). The survival of 12 Gy-irradiated mice after i.v. COMP-Ang1 treatment increased from a mean of 220 ± 9 hrs (median 216 hrs) to 258 ± 11 hrs (median 260 hrs). Thus, i.v. treatment with COMP-Ang1 could be very effective for suppressing side effects of radiation therapy in the GI tract by protecting against micro vascular endothelial apoptosis in intestinal villi.

## Claims

1. A coiled coil chimeric molecule comprising a coiled-coil domain of cartilage matrix protein (CMP), cartilage oligomeric matrix protein (COMP) or GCN4 linked to a receptor binding domain of angiopoietin-1 (Ang-1).

2. The coiled coil chimeric molecule of claim 1, wherein the receptor binding domain binds to Tie2 or Tie1 receptor.

3. The coiled coil chimeric molecule of claim 1, wherein the receptor binding domain of angiopoietin-1 is a fibrinogen-like domain.

4. The coiled coil chimeric molecule of claim 1, wherein the coiled coil domain is linked to an extracellular domain of the receptor.

5. An isolated nucleic acid encoding the coiled coil chimeric molecule of claim 1.

6. An expression vector comprising the nucleic acid of claim 5.

7. A isolated host cell comprising the vector of claim 6.

8. A soluble biologically active multimer comprising the coiled coil chimeric molecule according to claim 1, 2 or 3.

9. The multimer according to claim 8, which is a homomer.

10. The multimer according to claim 8, which is a heteromer.

11. The multimer according to claim 10, wherein the coiled coil domain is heterogeneous.

12. The multimer according to claim 8, which is a dimer, trimer, tetramer, pentamer, hexamer, heptamer, octamer, nanomer or decamer.

13. The multimer according to claim 8, wherein said coiled coil domains form parallel or anti-parallel structure.

14. A in vitro method of promoting cell growth comprising contacting the coiled coil chimeric molecule of claim 1, to a population of cells that express Tie2 receptors, which results in cell growth promotion.

15. The method according to claim 14, wherein said cells are endothelial calls, hematopoietic cells or other cells that express each specific receptor.

16. A in vitro method of promoting cell proliferation comprising contacting the coiled coil chimeric molecule of claim 1, to a population of cells that express Tie2 receptors, which results in cell proliferation,

17. The method according to claim 16, wherein said cells are endothelial cells, hematopoietic cells or other cells that express each specific receptor.

18. A in vitro method of decreasing or inhibiting cell proliferation comprising contacting the coiled coil chimeric molecule of claim 1, to a population of cells that express ligands that are specific for the receptor, wherein a multimeric form of the coiled coil chimeric molecule interacts with the ligand, which results in decrease or inhibition of cell proliferation.

19. The method according to claim 18, wherein said cells are endothelial cells, hematopoietic cells or other cells that express each specific receptor.

20. A in vitro method of decreasing or inhibiting ligand activity comprising contacting the coiled coil chimeric molecule of claim 1, to a sample comprising a ligand that is specific for the receptor, wherein a multimeric form of the coiled coil chimeric molecule binds the ligand, which results in a decrease or inhibition of ligand activity.

21. A method of making a chimeric molecule comprising:
(A) recombinantly combining a nucleic acid encoding a coiled-coil domain of cartilage matrix protein (CMP), cartilage oligomeric matrix protein (COMP) or GCN4 with a nucleic acid encoding a receptor binding region of angiopoietin-1 (Ang-1) to produce a chimeric gene construct; and
(B) expressing the gene construct in a host cell to produce the chimeric molecule.

22. Use of the molecule according to claim 1 for the manufacture of a medicament for protecting endothelial cells against radiation damage to a mammal that has been exposed to irradiation.

## Patentansprüche

1. Ein chimäres Doppelwendelmolekül, umfassend eine Doppelwendeldomäne des Knorpelmatrixproteins (CMP), oligomeren Knorpelmatrixproteins (COMP) oder GCN4, verbunden mit einer Rezeptor-Bindungsdomäne von Angiopoletin-1 (Ang-1).

2. Das chimäre Doppelwendelmolekül des Anspruchs 1, wobei die Rezeptor-Bindungsdomäne an Tie2 oder Tie1 Rezeptor bindet.

3. Das chimäre Doppelwendelmolekül des Anspruchs 1, wobei die Rezeptor-Bindungsdomäne von Angiopoietin-1 eine fibrinogenartige Domäne ist.

4. Das chimäre Doppelwendelmolekül des Anspruchs 1, wobei die Doppelwendeldomäne mit einer extrazelluläre Domäne des Rezeptor verbunden ist.

5. Eine isolierte Nukleinsäure, die das chimäre Doppelwendelmolekül des Anspruchs 1 kodiert.

6. Ein Expressionsvektor, der die Nukleinsäure des Anspruchs 5 umfasst.

7. Eine isolierte Wirtszelle, die den Vektor des Anspruchs 6 umfasst.

8. Ein lösliches, biologisch aktives Multimer, welches das chimäre Doppelwendelmolekül gemäß Anspruch 1, 2 oder 3 umfasst.

9. Multimer gemäß Anspruch 8, das ein Homomer ist.

10. Multimer gemäß Anspruch 8, das ein Heteromer ist.

11. Multimer gemäß Anspruch 10, wobei die Doppelwendeldomäne heterogen ist.

12. Multimer gemäß Anspruch 8, das ein Dimer, Trimer, Tetramer, Pentamer, Hexamer, Heptamer, Oktamer, Nanomer oder Dekamer ist.

13. Multimer gemäß Anspruch 8, wobei die Doppelwendeldomänen parallele oder antiparallele Struktur bilden.

14. Ein *in vitro*-Verfahren zur Förderung von Zellwachstum, umfassend: Kontaktieren des chimären Doppelwendelmoleküls des Anspruchs 1 mit einer Population von Zellen, welche Tie2 Rezeptoren exprimieren, woraus Förderung des Zellwachstums folgt.

15. Verfahren gemäß Anspruch 14, wobei die Zellen Endothelzellen, blutbildende Zellen oder andere Zellen sind, welche jeden spezifische Rezeptor exprimieren.

16. Ein *in vitro*-Verfahren zur Förderung von Zellvermehrung, umfassend: Kontaktieren des chimären Doppelwendelmoleküls des Anspruchs 1 mit einer Population von Zellen, welche Tie2 Rezeptoren exprimieren, woraus Zellvermehrung folgt.

17. Verfahren gemäß Anspruch 16, wobei die Zellen Endothelzellen, blutbildende Zellen oder andere Zellen sind, welche jeden spezifischen Rezeptor exprimieren.

18. Ein *in vitro*-Verfahren zur Senkung oder Hemmung von Zellvermehrung, umfassend:
Kontaktieren des chimären Doppelwendelmoleküls des Anspruchs 1, mit einer Population von Zellen, welche Liganden exprimieren, die spezifisch für den Rezeptor sind, wobei eine multimere Form des chimären Doppelwendelmoleküls mit dem Liganden wechselwirkt, woraus Senkung oder Hemmung von Zellvermehrung folgt.

19. Verfahren gemäß Anspruch 18, wobei die Zellen Endothelzellen, blutbildende Zellen oder andere Zellen sind, welche jeden spezifischen Rezeptor exprimieren.

20. Ein *in vitro*-Verfahren zur Senkung oder Hemmung von Ligandenaktivität umfassend:
Kontaktieren des chimären Doppelwendelmoleküls des Anspruchs 1 mit einer Probe, die einen Liganden beinhaltet, welcher spezifisch für den Rezeptor ist, wobei eine multimere Form des chimären Doppelwendelmoleküls den Liganden bindet, woraus eine Senkung oder Hemmung von Ligandenaktivität folgt.

21. Verfahren zur Herstellung eines chimären Moleküls, umfassend:
(A) rekombinantes Verbinden einer Nukleinsäure, die eine Doppelwendeldomäne des Knorpelmatrixproteins (CMP), oligomeren Knorpelmatrixproteins (COMP) oder GCN4 mit einer Nukleinsäure, welche einen Rezeptorbindungsbereich von Angiopoietin-1 (Ang-1) kodiert, um ein chimäres Genkonstrukt herzustellen; und
(B) Exprimieren des Genkonstrukts in einer Wirtszelle, um das chimäre Molekül herzustellen.

22. Verwendung des Moleküls gemäß Anspruch 1 für die Herstellung eines Medikaments, um Endothelzellen gegen Strahlungsschaden an einem Säugetier, welches Bestrahlung ausgesetzt wurde, zu schützen.

## Revendications

1. Molécule chimérique à superhélice comprenant un domaine de superhélice d'une protéine de matrice de cartilage (CMP), d'une protéine de matrice oligomère de cartilage (COMP) ou GCN4 liée à un domaine de liaison à un récepteur d'angiopoïétine-1 (Ang-1).

2. Molécule chimérique à superhélice selon la revendication 1, dans laquelle le domaine de liaison à un récepteur se lie au récepteur Tie2 ou Tie1.

3. Molécule chimérique à superhélice selon la revendication 1, dans laquelle le domaine de liaison à un récepteur de l'angiopoïétine-1 est un domaine de type fibrinogènc.

4. Molécule chimérique à superhélice selon la revendication 1, dans laquelle le domaine de superhélice est lié à un domaine extracellulaire du récepteur.

5. Acide nucléique isolé codant la molécule chimérique à superhélice de la revendication 1.

6. Vecteur d'expression comprenant l'acide nucléique de la revendication 5.

7. Cellule hôte isolée comprenant le vecteur de la revendication 6.

8. Multimère biologiquement actif et soluble comprenant la molécule chimérique à superhélice selon la revendication 1, 2 ou 3.

9. Multimère selon la revendication 8, qui est un homomère.

10. Multimère selon la revendication 8, qui est un hétéromère.

11. Multimère selon la revendication 10, dans lequel le domaine de superhélice est hétérogène.

12. Multimère selon la revendication 8, qui est un dimère, trimère, tétramère, pentamère, hexambre, heptamère, octamère, nanomère ou décamère.

13. Multimère selon la revendication 8, dans lequel ledit domaine de superhélice forme une structure parallèle ou antiparallèle.

14. Procédé in vitro pour favoriser la croissance cellulaire comprenant la mise en contact de la molécule chimérique à superhélice de la revendication 1, avec une population de cellules qui expriment des récepteurs Tie2, ce qui permet de favoriser la croissance cellulaire.

15. Procédé selon la revendication 14, dans lequel lesdites cellules sont des cellules endothéliales, des cellules hématopoïétiques ou d'autres cellules qui expriment chaque récepteur spécifique.

16. Procédé in vitro pour favoriser la prolifération cellulaire comprenant la mise en contact de la molécule chimérique à superhélice de la revendication 1, avec une population de cellules qui expriment des récepteurs Tie2, ce qui conduit à une prolifération cellulaire.

17. Procédé selon la revendication 16, dans lequel lesdites cellules sont des cellules endothéliales, des cellules hématopoïétiques ou d'autres cellules qui expriment chaque récepteur spécifique.

18. Procédé in vitro pour diminuer ou inhiber la prolifération cellulaire comprenant la mise en contact de la molécule chimérique à superhélice de la revendication 1, avec une population de cellules qui expriment des ligands qui sont spécifiques du récepteur, où une forme multimère de la molécule chimérique à superhélice interagit avec le ligand, ce qui conduit à une diminution ou une inhibition de la prolifération cellulaire.

19. Procédé selon la revendication 18, dans lequel lesdites cellules sont des cellules endothéliales, des cellules hématopoïétiques ou d'autres cellules qui expriment chaque récepteur spécifique.

20. Procédé in vitro pour diminuer ou inhiber l'activité d'un ligand comprenant la mise en contact de la molécule chimérique à superhélice de la revendication 1, avec un échanlillon comprenant un ligand qui est spécifique du récepteur, où une forme multimère de la molécule chimérique à superhélice se lie au ligand, ce qui conduit à une diminution ou une inhibition de l'activité de ligand.

21. Procédé de préparation d'une molécule chimérique comprenant les étapes consistant à :
(A) combiner de façon recombinante un acide nucléique codant un domaine de superhélice de la protéine de matrice de cartilage (CMP), de la protéine de matrice oligomère de cartilage (COMP) ou de GCN4 avec un acide nucléique codant une région de liaison à un récepteur d'angiopoïétine-1 (Ang-1), pour produire une construction génique chimérique ; et
(B) exprimer la construction génique dans une cellule hôte pour produire la molécule chimérique.

22. Utilisation de la molécule selon la revendication 1, pour la fabrication d'un médicament destiné à protéger des cellules endothéliales vis-à-vis d'un endomnagement par rayonnement chez un mammifère qui a été exposé à une irradiation.


### SEQUENCE LISTING

<110> KOH, Gou Young
<120> CHIMERIC COILED COIL MOLECULES
<130> 10010-01PCT
<160> 30
<170> PatentIn version 3.2
<210> 1
   <211> 786
   <212> DNA
   <213> Homo sapiens
<400> 1
   atgtctgcac ttctgatcct agctcttgtt ggagctgcag ttgctgacta caaagacgat      60
   gacgacaagc ttgtcaatct ttgcactaaa gaaggtgttt tactaaaggg aggaaaaaga     120
   gaggaagaga aaccatttag agactgtgca gatgtatatc aagctggttt taataaaagt     180
   ggaatctaca ctatttatat taataatatg ccagaaccca aaaaggtgtt ttgcaatatg     240
   gatgtcaatg ggggaggttg gactgtaata caacatcgtg aagatggaag tctagatttc     300
   caaagaggct ggaaggaata taaaatgggt tttggaaatc cctccggtga atattggctg     360
   gggaatgagt ttatttttgc cattaccagt cagaggcagt acatgctaag aattgagtta     420
   atggactggg aagggaaccg agcctattca cagtatgaca gattccacat aggaaatgaa     480
   aagcaaaact ataggttgta tttaaaaggt cacactggga cagcaggaaa acagagcagc     540
   ctgatcttac acggtgctga tttcagcact aaagatgctg ataatgacaa ctgtatgtgc     600
   aaatgtgccc tcatgttaac aggaggatgg tggtttgatg cttgtggccc ctccaatcta     660
   aatggaatgt tctatactgc gggacaaaac catggaaaac tgaatgggat aaagtggcac     720
   tacttcaaag ggcccagtta ctccttacgt tccacaacta tgatgattcg acctttagat     780
   ttttga                                                                786
<210> 2
   <211> 260
   <212> PRT
   <213> Homo sapiens
<400> 2
<210> 3
   <211> 897
   <212> DNA
   <213> Homo sapiens
<400> 3
   atgaagacga tcatcgccct gagctacatc ttctgcctgg tattcgccga ctacaaggac     60
   gatgatgaca aggggatctt aatgaaacag ctggaagaca aagttgaaga actgctgtct    120
   aaaaactacc acctggaaaa cgaagttgct cgtctgaaaa aactggttgg tgaaggatcc    180
   cttgtcaatc tttgcactaa agaaggtgtt ttactaaagg gaggaaaaag agaggaagag    240
   aaaccattta gagactgtgc agatgtatat caagctggtt ttaataaaag tggaatctac    300
   actatttata ttaataatat gccagaaccc aaaaaggtgt tttgcaatat ggatgtcaat    360
   gggggaggtt ggactgtaat acaacatcgt gaagatggaa gtctagattt ccaaagaggc    420
   tggaaggaat ataaaatggg ttttggaaat ccctccggtg aatattggct ggggaatgag    480
   tttatttttg ccattaccag tcagaggcag tacatgctaa gaattgagtt aatggactgg    540
   gaagggaacc gagcctattc acagtatgac agattccaca taggaaatga aaagcaaaac    600
   tataggttgt atttaaaagg tcacactggg acagcaggaa aacagagcag cctgatctta    660
   cacggtgctg atttcagcac taaagatgct gataatgaca actgtatgtg caaatgtgcc    720
   ctcatgttaa caggaggatg gtggtttgat gcttgtggcc cctccaatct aaatggaatg    780
   ttctatactg cgggacaaaa ccatggaaaa ctgaatggga taaagtggca ctacttcaaa    840
   gggcccagtt actccttacg ttccacaact atgatgattc gacctttaga tttttga       897
<210> 4
   <211> 298
   <212> PRT
   <213> Homo sapiens
<400> 4
   Met Lys Thr Ile Ile Ala Leu Ser Tyr Ile Phe Cys Leu Val Phe Ala
   1               5                   10                  15
   Asp Tyr Lys Asp Asp Asp Asp Lys Gly Ile Leu Met Lys Gln Leu Glu
               20                  25                  30
   Asp Lys Val Glu Glu Leu Leu Ser Lys Asn Tyr His Leu Glu Asn Glu
           35                  40                  45
   Val Ala Arg Leu Lys Lys Leu Val Gly Glu Gly Ser Leu Val Asn Leu
       50                  55                  60
   Cys Thr Lys Glu Gly Val Leu Leu Lys Gly Gly Lys Arg Glu Glu Glu
   65                  70                  75                  80
   Lys Pro Phe Arg Asp Cys Ala Asp Val Tyr Gln Ala Gly Phe Asn Lys
                   85                  90                  95
   Ser Gly Ile Tyr Thr Ile Tyr Ile Asn Asn Met Pro Glu Pro Lys Lys
               100                 105                 110
   Val Phe Cys Asn Met Asp Val Asn Gly Gly Gly Trp Thr Val Ile Gln
           115                 120                 125
   His Arg Glu Asp Gly Ser Leu Asp Phe Gln Arg Gly Trp Lys Glu Tyr
       130                 135                 140
   Lys Met Gly Phe Gly Asn Pro Ser Gly Glu Tyr Trp Leu Gly Asn Glu
   145                 150                 155                 160
   Phe Ile Phe Ala Ile Tyr Ser Gln Arg Gln Tyr Met Leu Arg Ile Glu
                   165                 170                 175
   Leu Met Asp Trp Glu Gly Asn Arg Ala Tyr Ser Gln Tyr Asp Arg Phe
               180                 185                 190
   His Ile Gly Asn Glu Lys Gln Asn Tyr Arg Leu Tyr Leu Lys Gly His
           195                 200                 205
   Thr Gly Tyr Ala Gly Lys Gln Ser Ser Leu Ile Leu His Gly Ala Asp
       210                 215                 220
   Phe Ser Thr Lys Asp Ala Asp Asn Asp Asn Cys Met Cys Lys Cys Ala
   225                 230                 235                 240
   Leu Met Leu Thr Gly Gly Trp Trp Phe Asp Ala Cys Gly Pro Ser Asn
                   245                 250                 255
   Leu Asn Gly Met Phe Tyr Thr Ala Gly Gln Asn His Gly Lys Leu Asn
               260                 265                 270
   Gly Ile Lys Trp His Tyr Phe Lys Gly Pro Ser Tyr Ser Leu Arg Ser
           275                 280                 285
   Thr Thr Met Met Ile Arg Pro Leu Asp Phe
       290                 295
<210> 5
   <211> 933
   <212> DNA
   <213> Homo sapiens
<400> 5
   atgaagacga tcatcgccct gagctacatc ttctgcctgg tattcgccga ctacaaggac      60
   gatgatgaca aggggatctt agatgaagat ccgtgcgaat ccaaaagcat agtgaaattc     120
   cagaccaaag tggaagaact gatcaacacc ctgcagcaga aactggaagc ggtggcgaaa     180
   cgtatcgaag cgctggaaaa caaaatcatc ggatcccttg tcaatctttg cactaaagaa     240
   ggtgttttac taaagggagg aaaaagagag gaagagaaac catttagaga ctgtgcagat     300
   gtatatcaag ctggttttaa taaaagtgga atctacacta tttatattaa taatatgcca     360
   gaacccaaaa aggtgttttg caatatggat gtcaatgggg gaggttggac tgtaatacaa     420
   catcgtgaag atggaagtct agatttccaa agaggctgga aggaatataa aatgggtttt     480
   ggaaatccct ccggtgaata ttggctgggg aatgagttta tttttgccat taccagtcag     540
   aggcagtaca tgctaagaat tgagttaatg gactgggaag ggaaccgagc ctattcacag     600
   tatgacagat tccacatagg aaatgaaaag caaaactata ggttgtattt aaaaggtcac     660
   actgggacag caggaaaaca gagcagcctg atcttacacg gtgctgattt cagcactaaa     720
   gatgctgata atgacaactg tatgtgcaaa tgtgccctca tgttaacagg aggatggtgg     780
   tttgatgctt gtggcccctc caatctaaat ggaatgttct atactgcggg acaaaaccat     840
   ggaaaactga atgggataaa gtggcactac ttcaaagggc ccagttactc cttacgttcc     900
   acaactatga tgattcgacc tttagatttt tga                                  933
<210> 6
   <211> 309
   <212> PRT
   <213> Homo sapiens
<400> 6
   Met Lys Thr Ile Ile Leu Ser Tyr Ile Phe Cys Leu Val Phe Ala Asp
   1               5                   10                  15
   Tyr Lys Asp Asp Asp Asp Lys Gly Ile Leu Asp Glu Asp Pro Cys Glu
               20                  25                  30
   Cys Lys Ser Ile Val Lys Phe Gln Thr Lys Val Glu Glu Leu Ile Asn
           35                  40                  45
   Thr Leu Gln Gln Lys Leu Glu Ala Val Ala Lys Arg Ile Glu Ala Leu
       50                  55                  60
   Glu Asn Lys Ile Ile Gly Ser Leu Val Asn Leu Cys Thr Lys Glu Gly
   65                  70                  75                  80
   Val Leu Leu Lys Gly Gly Lys Arg Glu Glu Glu Lys Pro Phe Arg Asp
                   85                  90                  95
   Cys Ala Asp Val Tyr Gln Ala Gly Phe Asn Lys Ser Gly Ile Tyr Thr
               100                 105                 110
   Ile Tyr Ile Asn Asn Met Pro Glu Pro Lys Lys Val Phe Cys Asn Met
           115                 120                 125
   Asp Val Asn Gly Gly Gly Trp Thr Val Ile Gln His Arg Glu Asp Gly
       130                 135                 140
   Ser Leu Asp Phe Gln Arg Gly Trp Lys Glu Tyr Lys Met Gly Phe Gly
   145                 150                 155                 160
   Asn Pro Ser Gly Glu Tyr Trp Leu Gly Asn Glu Phe Ile Phe Ala Ile
                   165                 170                 175
   Tyr Ser Gln Arg Gln Tyr Met Leu Arg Ile Glu Leu Met Asp Trp Glu
               180                 185                 190
   Gly Asn Arg Ala Tyr Ser Gln Tyr Asp Arg Phe His Ile Gly Asn Glu
           195                 200                 205
   Lys Gln Asn Tyr Arg Leu Tyr Leu Lys Gly His Thr Gly Thr Ala Gly
       210                 215                 220
   Lys Gln Ser Ser Leu Ile Leu His Gly Ala Asp Phe Ser Thr Lys Asp
   225                 230                 235                 240
   Ala Asp Asn Asp Asn Cys Met Cys Lys Cys Ala Leu Met Leu Thr Gly
                   245                 250                 255
   Gly Trp Trp Phe Asp Ala Cys Gly Pro Ser Asn Leu Asn Gly Met Phe
               260                 265                 270
   Tyr Thr Ala Gly Gln Asn His Gly Lys Leu Asn Gly Ile Lys Trp His
           275                 280                 285
   Tyr Phe Lys Gly Pro Ser Tyr Ser Leu Arg Ser Thr Thr Met Met Ile
       290                 295                 300
   Arg Pro Leu Asp Phe
   305
<210> 7
   <211> 939
   <212> DNA
   <213> Homo sapiens
<400> 7
   atgaagacga tcatcgccct gagctacatc ttctgcctgg tattcgccga ctacaaggac      60
   gatgatgaca aggggatctt agacctagcc ccacagatgc ttcgagaact ccaggagact     120
   aatgcggcgc tgcaagacgt gagagagctc ttgcgacagc aggtcaagga gatcaccttc     180
   ctgaagaata cggtgatgga atgtgacgct tgcggaggat cccttgtcaa tctttgcact     240
   aaagaaggtg ttttactaaa gggaggaaaa agagaggaag agaaaccatt tagagactgt     300
   gcagatgtat atcaagctgg ttttaataaa agtggaatct acactattta tattaataat     360
   atgccagaac ccaaaaaggt gttttgcaat atggatgtca atgggggagg ttggactgta     420
   atacaacatc gtgaagatgg aagtctagat ttccaaagag gctggaagga atataaaatg     480
   ggttttggaa atccctccgg tgaatattgg ctggggaatg agtttatttt tgccattacc     540
   agtcagaggc agtacatgct aagaattgag ttaatggact gggaagggaa ccgagcctat     600
   tcacagtatg acagattcca cataggaaat gaaaagcaaa actataggtt gtatttaaaa     660
   ggtcacactg ggacagcagg aaaacagagc agcctgatct tacacggtgc tgatttcagc     720
   actaaagatg ctgataatga caactgtatg tgcaaatgtg ccctcatgtt aacaggagga     780
   tggtggtttg atgcttgtgg cccctccaat ctaaatggaa tgttctatac tgcgggacaa     840
   aaccatggaa aactgaatgg gataaagtgg cactacttca aagggcccag ttactcctta     900
   cgttccacaa ctatgatgat tcgaccttta gatttttga                            939
<210> 8
   <211> 312
   <212> PRT
   <213> Homo sapiens
<400> 8
   Met Lys Thr Ile Ile Ala Leu Ser Tyr Ile Phe Cys Leu Val Phe Ala
   1               5                   10                  15
   Asp Tyr Lys Asp Asp Asp Asp Lys Gly Ile Leu Asp Leu Ala Pro Gln
               20                  25                  30
   Met Leu Arg Glu Leu Gln Glu Thr Asn Ala Ala Leu Gln Asp Val Arg
           35                  40                  45
   Glu Leu Leu Arg Gln Gln Val Lys Glu Ile Thr Phe Leu Lys Asn Thr
       50                  55                  60
   Val Met Glu Cys Asp Ala Cys Gly Gly Ser Leu Val Asn Leu Cys Thr
   65                  70                  75                  80
   Lys Glu Gly Val Leu Leu Lys Gly Gly Lys Arg Glu Glu Glu Lys Pro
                   85                  90                  95
   Phe Arg Asp Cys Ala Asp Val Tyr Gln Ala Gly Phe Asn Lys Ser Gly
               100                 105                 110
   Ile Tyr Thr Ile Tyr Ile Asn Asn Met Pro Glu Pro Lys Lys Val Phe
           115                 120                 125
   Cys Asn Met Asp Val Asn Gly Gly Gly Trp Thr Val Ile Gln His Arg
       130                 135                 140
   Glu Asp Gly Ser Leu Asp Phe Gln Arg Gly Trp Lys Glu Tyr Lys Met
   145                 150                 155                 160
   Gly Phe Gly Asn Pro Ser Gly Glu Tyr Trp Leu Gly Asn Glu Phe Ile
                   165                 170                 175
   Phe Ala Ile Tyr Ser Gln Arg Gln Tyr Met Leu Arg Ile Glu Leu Met
               180                 185                 190
   Asp Trp Glu Gly Asn Arg Ala Tyr Ser Gln Tyr Asp Arg Phe His Ile
           195                 200                 205
   Gly Asn Glu Lys Gln Asn Tyr Arg Leu Tyr Leu Lys Gly His Thr Gly
       210                 215                 220
   Thr Ala Gly Lys Gln Ser Ser Leu Ile Leu His Gly Ala Asp Phe Ser
   225                 230                 235                 240
   Thr Lys Asp Ala Asp Asn Asp Asn Cys Met Cys Lys Cys Ala Leu Met
                   245                 250                 255
   Leu Thr Gly Gly Trp Trp Phe Asp Ala Cys Gly Pro Ser Asn Leu Asn
   260 265 270
   Gly Met Phe Tyr Thr Ala Gly Gln Asn His Gly Lys Leu Asn Gly Ile
   275 280 285
   Lys Trp His Tyr Phe Lys Gly Pro Ser Tyr Ser Leu Arg Ser Thr Thr
   290 295 300
   Met Met Ile Arg Pro Leu Asp Phe
   305 310
<210> 9
   <211> 25
   <212> DNA
   <213> Artificial
<220>
   <223> Primer for human Ang 1
<400> 9
   gtgcggattc acaatgacag ttttc 25
<210> 10
   <211> 25
   <212> DNA
   <213> Artificial
<220>
   <223> Primer for human Ang 1
<400> 10
   gtgcggcagt acaatgacag ttttc 25
<210> 11
   <211> 25
   <212> DNA
   <213> Artificial
<220>
   <223> Primer for human Ang 1
<400> 11
   gctttcagat atctaaaggt cgaat 25
<210> 12
   <211> 25
   <212> DNA
   <213> Artificial
<220>
   <223> Primer for human Ang 1
<400> 12
   gctttcaaaa atctaaaggt cgaat 25
<210> 13
   <211> 23
   <212> DNA
   <213> Artificial
<220>
   <223> Primer for human Ang 1
<400> 13
   cagaaaagct tgggagaaga tat 23
<210> 14
   <211> 22
   <212> DNA
   <213> Artificial
<220>
   <223> Primer for human Ang 1
<400> 14
   tagaaggcac agtcgaggct ga 22
<210> 15
   <211> 29
   <212> DNA
   <213> Artificial
<220>
   <223> Primer for GCN4
<400> 15
   cagatcttaa tgaaacagct ggaagacaa 29
<210> 16
   <211> 31
   <212> DNA
   <213> Artificial
<220>
   <223> Primer for GCN4
<400> 16
   ttggatcctt caccaaccag ttttttcaga c 31
<210> 17
   <211> 30
   <212> DNA
   <213> Artificial
<220>
   <223> Primer for CMP
<400> 17
   ccagatctta gaagaagatc cgtgcgaatg 30
<210> 18
   <211> 29
   <212> DNA
   <213> Artificial
<220>
   <223> Primer for CMP
<400> 18
   aaggatccga tgattttgtt ttccagcgc 29
<210> 19
   <211> 30
   <212> DNA
   <213> Artificial
<220>
   <223> Primer for COMP
<400> 19
   ccagatctta gacctagccc cacagatgct 30
<210> 20
   <211> 30
   <212> DNA
   <213> Artificial
<220>
   <223> Primer for COMP
<400> 20
   ttggatcctc cgcaagcgtc acattccatc 30
<210> 21
   <211> 109
   <212> DNA
   <213> Artificial
<220>
   <223> Primer for SHG-FLAG
<400> 21
   aagcttaagc ttgccaccat gaagacgatc atcgccctga gctacatctt ctgcctggta 60
   ttcgccgact acaaggacga tgatgacaag gggatccact agtctcgag 109
<210> 22
   <211> 109
   <212> DNA
   <213> Artificial
<220>
   <223> Primer for SHG-FLAG
<400> 22
   ctcgagacta gtggatcccc ttgtcatcat cgtccttgta gtcggcgaat accaggcaga 60
   agatgtagct cagggcgatg atcgtcttca tggtggcaag cttaagctt 109
<210> 23
   <211> 30
   <212> DNA
   <213> Artificial
<220>
   <223> Primer for A1LF
<400> 23
   ttggatccct tgtcaatctt tgcactaaag 30
<210> 24
   <211> 33
   <212> DNA
   <213> Artificial
<220>
   <223> Primer for A1LF
<400> 24
   ttctcgagtc aaaaatctaa aggtcgaatc atc 33
<210> 25
   <211> 14
   <212> PRT
   <213> Artificial
<220>
   <223> Preprotrypsin leader sequence for Native Ang 1
<400> 25
   Met Ser Ala Leu Leu Ile Leu Ala Leu Val Gly Ala Ala Ala
   1 5 10
<210> 26
   <211> 8
   <212> PRT
   <213> Artificial
<220>
   <223> FLAG tag sequence
<400> 26
   Asp Tyr Lys Asp Asp Asp Asp Lys
   1 5
<210> 27
   <211> 16
   <212> PRT
   <213> Artificial
<220>
   <223> bridging sequence
<400> 27
   Asp Ile Gln His Ser Gly Gly Arg Ser Ser Leu Glu Gly Pro Arg Phe
   1 5 10 15
<210> 28
   <211> 10
   <212> PRT
   <213> Artificial
<220>
   <223> Myc epitope
<400> 28
   Glu Gln Lys Leu Ile Ser Glu Glu Asp Leu
   1 5 10
<210> 29
   <211> 5
   <212> PRT
   <213> Artificial
<220>
   <223> short bridging sequence
<400> 29
   Asn Met His Thr Gly
   1 5
<210> 30
   <211> 6
   <212> PRT
   <213> Artificial
<220>
   <223> His-Tag sequence
<400> 30
   His His His His His His
   1 5